# EUROPEAN PATENT SPECIFICATION

(11) **EP 4 419 058 B1**
(45) Date of publication and mention of the grant of the patent: **11.11.2026**
(21) Application number: 21805352.8
(22) Date of filing: 21.10.2021
(51) Int. Cl.: A61F 13/49

(54) **PANT-TYPE WEARABLE ARTICLE**
HÖSCHENARTIGER WEARABLE-ARTIKEL
ARTICLE À PORTER DE TYPE CULOTTE

(43) Date of publication of application: 28.08.2024
(73) Proprietor: The Procter & Gamble Company, Cincinnati, OH 45202 (US)
(72) Inventor: CHENG, Chunmin, Beijing 101312 (CN); MORIMOTO, Koichi, Beijing 101312 (CN); STILLWAGON, Mark Thomas, Cincinnati, Ohio 45202 (US); YIN, Qilei, Beijing 101312 (CN); MISHRA, Piyush, 53881 Euskirchen (DE)
(74) Representative: P&G Patent Germany
(86) International application number: PCT/CN2021/125242
(87) International publication number: WO 2023/065207

(56) References cited:
- WO-A1-2020/164379
- AU-A1- 2018 258 551
- JP-B1- 6 074 523
- US-A1- 2020 260 798
- US-A1- 2020 260 799
- US-A1- 2020 261 281
- US-A1- 2021 267 818

## Description

### FIELD OF THE INVENTION

The present invention relates to pant-type wearable articles having an elastic belt with, improved softness, and improved function intuitive appearance.

### BACKGROUND OF THE INVENTION

Infants and other incontinent individuals wear absorbent articles such as diapers to receive and contain urine and other body exudates. Pull-on absorbent articles, or pant-type absorbent articles, are those which are donned by inserting the wearer's legs into the leg openings and sliding the article up into position about the lower torso. Pant-type absorbent articles have become popular for use on children who are able to walk and often who are toilet training, as well as for younger children who become more active in movement such that application of taped-type absorbent articles tends to be more difficult, and also for younger babies requiring a soft fit around the waist opening and leg openings.

Pant-type articles may take various structures wherein the circumference of the waist opening and vicinity thereof is made elastic enough to facilitate the wearer or the caregiver to expand the article and insert the wearer's legs into the leg openings for wearing the article. The region of the waist circumference and vicinity thereof is often referred to as the elastic belt. One type of structure for the pant-type article is the belt-type pant having a central chassis to cover the crotch region of the wearer and a separate elastic belt defining the waist opening and leg opening, such as described in PCT Publication WO 2006/17718A. Another type of structure for the pant-type article is the uni-body pant configured such that the outer cover of the article completely covers the entirety of the garment-facing surface of the article, wherein the portion configured to stretch about the torso is considered the elastic belt region.

Whatever the structure of the pant-type article may be, pant-type articles provide only a very small range of size adjustment or body configuration adjustment based on the structural limitations of the article. As such, pant-type articles are typically so configured to accommodate size and configuration ranges by providing the elastic belt region very stretchable and comfortable to wear, yet with reliable fit such that sufficient protection against sagging and leakage may be provided. Further, the elastic belt region may be the portion which is most touched and observed by the wearer or the caregiver upon use, and thus its properties most associated with the function and quality of the article. An appearance that intuitively connotes the function of the article, or the function of a particular part of the article is advantageous.

Based on the foregoing, there is a need for a wearable article providing softness, stretchability for ease of application, fit for preventing sagging, and comfort and improved breathability for skin health. There is also a need for a wearable article having function intuitive appearance while also being underwear like. There is also a need for providing such a wearable article which can be economically made.

US2020/261281 relates to a wearable article comprising a front and back elastic belt region. The laminate comprises an inner sheet, an outer sheet, and elastic strands running in a transverse direction. The laminate comprises an elastic bonding which continuously bonds the elastic member for at least about 10 mm in the direction of stretch in a region adjacent the side edges of the front and back elastic belts, and a vertical bonding applied to at least one of the inner sheet and the outer sheet with intervals in the transverse direction to intermittently bond the inner sheet and the outer sheet.

US2020/260799 discloses a wearable article with front and back elastic belt regions. The front and back elastic belt regions each comprise a laminate, an elastic bonding continuously bonding an elastic member in the direction of stretch, and a vertical bonding applied to at least one of inner and outer sheets to intermittently bond the elastic member to at least one of the inner and outer sheets.

WO2020/164379 concerns a wearable article comprising a front and back elastic belt region. The laminate comprises an inner and an outer sheet and a plurality of elastic strands running in the transverse direction. The laminate further comprises an elastic bonding which continuously bonds the elastic member for at least about 10 mm in the direction of stretch in a region adjacent the side edges of the front and back elastic belts, and a vertical bonding applied to at least one of the inner sheet and the outer sheet.

US2020/260798 discloses a wearable article comprising a front and back elastic belt. Certain of the front/back elastic belt is a non-elastic region; the front and back non-elastic regions have a First Belt Pattern and the crotch region has a First Crotch Pattern; wherein the First Belt Pattern as well as First Crotch Pattern is a Geometric Pattern.

### SUMMARY OF THE INVENTION

The present invention is directed to a wearable article as defined by claim 1 and being continuous in a longitudinal direction and a transverse direction comprising a front elastic belt region, a back elastic belt region, a crotch region, and a pair of side seams which join the front elastic belt region and the back elastic belt region to form a waist opening and a pair of leg openings; the crotch region extending longitudinally between the front elastic belt region and the back elastic belt region;
wherein each of the front and back elastic belt region comprises a laminate comprising an inner sheet, an outer sheet, and a plurality of elastic members running in the transverse direction, the longitudinal dimension between adjacent elastic members forming an elastic spacing;
wherein the laminate further comprises an elastic bonding which continuously bonds the elastic members for at least about 10mm in the direction of stretch in a region adjacent the side edges of the front and back elastic belt regions, and a plurality of discrete bond units disposed between the elastic bondings in the transverse direction, each discrete bond unit applied to at least one of the inner sheet and the outer sheet, wherein there is at least one discrete bond unit disposed in each elastic spacing,
the plurality of discrete bond units forms a plurality of longitudinal columns, each longitudinal column comprising a collection of longitudinal spacings;
the plurality of discrete bond units forms a plurality of transverse columns, each transverse column comprising a collection of transverse spacings,
wherein a collection of discrete bond units provided by at least 2 longitudinal columns and at least 2 transverse columns form a pattern of discrete bond units, wherein the laminate comprises at least a first pattern and a second pattern of discrete bond units, the first pattern and the second pattern of discrete bond units having a difference in one or more of: the longitudinal dimension, the transverse dimension, the collection of longitudinal spacings, and the collection of transverse spacings.

The present invention is also directed to packages comprising such wearable article, and method of making such wearable article.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:
Figure 1A is a perspective view of one embodiment of a wearable article of the present invention.
Figure 1B is a schematic view of one embodiment of a wearable article of the present invention in a contracted state showing the front side of the article.
Figure 2 is a schematic plan view of one embodiment of a wearable article of the present invention with the seams unjoined and in a flat uncontracted condition showing the garment facing surface.
Figure 3A is a schematic plan view of one embodiment of a laminate of the present invention showing the elastic member positioning, elastic adhesive bondings and area where patterns of discrete bond units are disposed.
Figure 3B is another embodiment similar to Figure 3A with different patterns of discrete bond units.
Figure 3C is another embodiment similar to Figure 3A with different patterns of discrete bond units.
Figure 3D is another embodiment of a laminate seamed with the laminate of Figure 3A to form an elastic belt.
Figure 3E is an expanded schematic plan view of Figure 3A.
Figure 3F is an expanded schematic plan view of Figure 3B.
Figure 3G is another embodiment similar to Figure 3A with different patterns of discrete bond units.
Figure 4A is a perspective view of an adhesive application apparatus positioned adjacent to an advancing sheet.
Figure 4B is a perspective view of an embodiment of a sheet carrier including a pattern roller having a continuous pattern surface and plurality of base surfaces.
Figure 4C is a schematic cross-sectional side view of a fluid application apparatus.
Figure 4D is a schematic cross-sectional side view of a laminate assembly apparatus including the adhesive apparatus of Figure 4A.
Figure 5 is a schematic view of an example of a hanger-type sample holding fixture according to the "Whole Article Force Measurement".
Figure 6A is an embodiment of an artwork provided on the wearable article of the present invention with the seams unjoined and in a flat uncontracted condition showing the garment facing surface.
Figure 6B is another embodiment of an artwork provided on the laminate of the present invention.

### DEFINITIONS

As used herein, the following terms shall have the meaning specified thereafter:
"Wearable article" refers to articles of wear which may be in the form of pants, taped diapers, incontinent briefs, feminine hygiene garments, and the like. The "wearable article" may be so configured to also absorb and contain various exudates such as urine, feces, and menses discharged from the body. The "wearable article" may serve as an outer cover adaptable to be joined with a separable disposable absorbent insert for providing absorbent and containment function, such as those disclosed in PCT publication WO 2011/087503A.
"Pant" refers to disposable absorbent articles having a pre-formed waist and leg openings. A pant may be donned by inserting a wearer's legs into the leg openings and sliding the pant into position about the wearer's lower torso. Pants are also commonly referred to as "closed diapers", "prefastened diapers", "pull-on diapers", "training pants" and "diaper-pants".
"Longitudinal" refers to a direction running substantially perpendicular from a waist edge to an opposing waist edge of the article and generally parallel to the maximum linear dimension of the article.
"Transverse" refers to a direction perpendicular to the longitudinal direction.
"Proximal" and "distal" refer respectively to the position closer or farther relative to the longitudinal center of the article.
"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).
"Disposed" refers to an element being located in a particular place or position.
"Joined" refers to configurations whereby an element is directly secured to another element by affixing the element directly to the other element and to configurations whereby an element is indirectly secured to another element by affixing the element to intermediate member(s) which in turn are affixed to the other element.
"Film" refers to a sheet-like material wherein the length and width of the material far exceed the thickness of the material. Typically, films have a thickness of about 0.5 mm or less.
"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".
"Extendibility" and "extensible" mean that the width or length of the component in a relaxed state can be extended or increased.
"Elasticated" and "elasticized" mean that a component comprises at least a portion made of elastic material.
"Elongatable material", "extensible material", or "stretchable material" are used interchangeably and refer to a material that, upon application of a biasing force, can stretch to an elongated length of at least about 110% of its relaxed, original length (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastomeric." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "substantially non-elastic" or "substantially non-elastomeric". For example, an elongatable material that has an initial length of 100mm can extend at least to 150mm, and upon removal of the force retracts to a length of at least 145mm (i.e., exhibiting a 10% recovery).
"Dimension", "Length", "Width", "Pitch", "Diameter", "Aspect Ratio", "Angle", and "Area" of the article are all measured in a state wherein the article is extended to the Full Stretch Circumference W1 according to the "Whole Article Force Measurement" herein, and utilizing a ruler or a loupe, unless specified otherwise.
"Artwork" refers to a visual presentation to the naked eye, which is provided by printing or otherwise, and having a color. Printing includes various methods and apparatus well known to those skilled in the art such as lithographic, screen printing, flexographic, and gravure ink jet printing techniques.
"Color" or "Colored" as referred to herein includes any primary color except color white, i.e., black, red, blue, violet, orange, yellow, green, and indigo as well as any declination thereof or mixture thereof. The color white is defined as those colors having a L* value of at least 94, an a* value equal to 0 ± 2, and a b* value equal to 0 ± 2 according to the CIE L* a* b* color system.

### DETAILED DESCRIPTION OF THE INVENTION

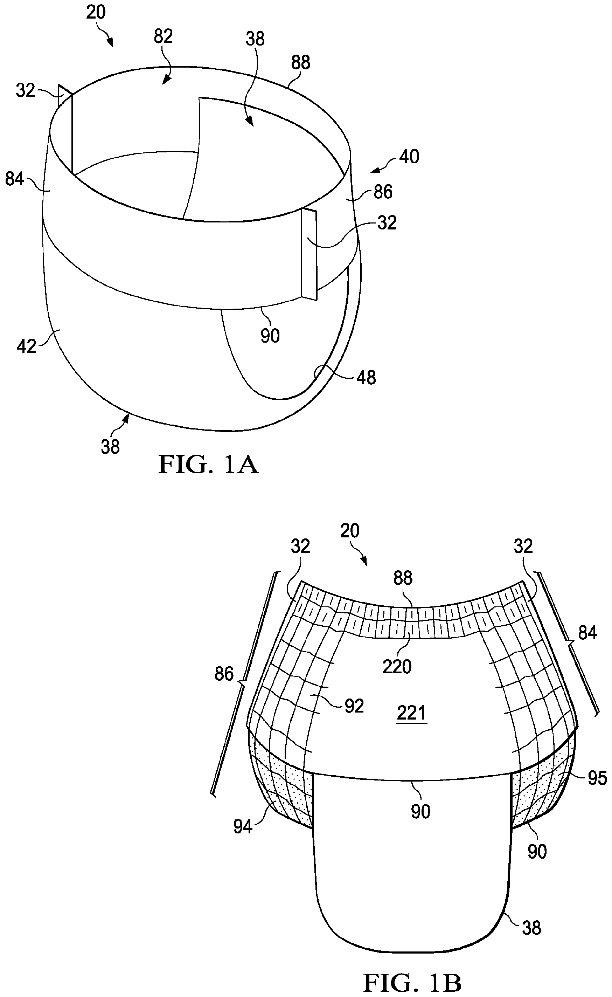

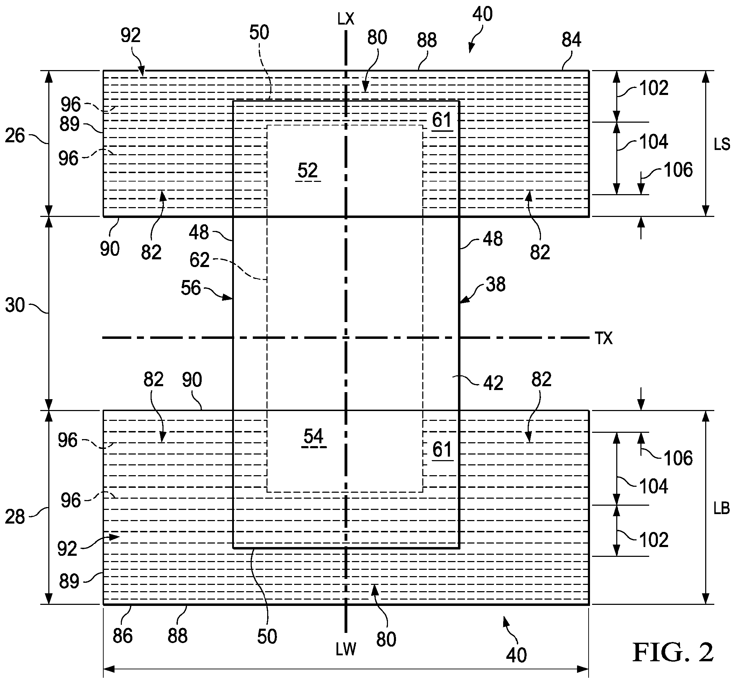

Figure 1A is a perspective view of a wearable article (20) of the present invention, Figure 1B is a schematic view of a wearable article of the present invention in a contracted state showing the front side, and Figure 2 is a schematic plan view of a wearable article with the seams unjoined and in its flat uncontracted condition showing the garment-facing surface. The wearable article (20) has a longitudinal centerline LX which also serves as the longitudinal axis, and a transverse centerline TX which also serves as the transverse axis. The wearable article (20) has a body facing surface, a garment facing surface, a front elastic belt region (84), a back elastic belt region (86), a crotch region (30), and side seams (32) which join the front elastic belt region (84) and the back elastic belt region (86), to form two leg openings and a waist opening.

The wearable article (20) may be a belt-type pant as in Figures 1A, 1B, and 2 comprising a central chassis (38) to cover the crotch region (30) of the wearer, a front elastic belt (84) and a back elastic belt (86) (hereinafter may be referred to as "front and back elastic belts"), the front and back elastic belts (84, 86) forming a discrete ring-like elastic belt (40) extending transversely defining the waist opening. For the belt-type pant, the discrete ring-like elastic belt (40) may also be referred to as the elastic belt (40). For the belt-type pant as in Figures 1A, 1B, and 2, the front and back elastic belts (84, 86) and the central chassis (38) jointly define the leg openings. For the belt-type pant, the front elastic belt (84) is the front region (26), and the back elastic belt (86) is the back region (28), and the remainder is the crotch region (30). While not shown, the wearable article (20) may be a uni-body type pant configured such that the outer cover of the central chassis (38) and the elastic belt (40) are common. For the uni-body type pant, the portion extending in the transverse direction between the side seams (32), respectively, are considered the front region (26) and the back region (28), and the remainder is the crotch region (30). For the uni-body type pant, the front region (26) is considered the front elastic belt region (84), and the back region (28) is considered the back elastic belt region (86).

The central chassis (38) may comprise a topsheet, a backsheet and an absorbent core (62) disposed between the topsheet and the backsheet, and further an outer cover layer (42) for covering the garment-facing side of the backsheet. The topsheet may be a water permeable substrate. The backsheet may be a water impermeable film. The outer cover layer (42) may be a nonwoven sheet. The central chassis (38) may contain an absorbent core (62) for absorbing and containing body exudates disposed on the central chassis (38), and an absorbent material non-existing region (61) surrounding the periphery of the absorbent core (62). The absorbent material non-existing region (61) may be made of the topsheet and/or the backsheet and/or the outer cover layer (42) and/or other parts configuring the central chassis (38). In the embodiment shown in Figure 2, the central chassis (38) has a generally rectangular shape, left and right longitudinally extending side edges (48) and front and back transversely extending end edges (50). The absorbent core (62) may exist through the entire longitudinal dimension of the crotch region and extending at least partly in the front region (26); or at least partly in both the front and back regions (26, 28). The central chassis (38) may have a front waist panel (52) positioned in the front region (26) of the absorbent article (20), a back waist panel (54) positioned in the back region (28), and a crotch panel (56) between the front and back waist panels (52, 54) in the crotch region (30). The center of the front elastic belt (84) is joined to a front waist panel (52) of the central chassis (38), the center of the back elastic belt (86) is joined to a back waist panel (54) of the central chassis (38), the front and back elastic belts (84, 86) each having a left side panel and a right side panel (82) where the central chassis (38) does not overlap. The central chassis has a crotch panel (56) positioned between the front waist panel (52) and the back waist panel (54).

The absorbent core (62) may include an absorbent layer and an acquisition layer. The absorbent layer is the region wherein absorbent materials having a high retention capacity, such as superabsorbent polymers, are present. The absorbent layer may be substantially cellulose free. Superabsorbent polymers of the absorbent layer may be disposed between first and second layers of material immobilized by a fibrous layer of thermoplastic adhesive material. The first and second layers of materials may be nonwoven fibrous webs including synthetic fibers, such as mono-constituent fibers of PE, PET and PP, multiconstituent fibers such as side by side, core/sheath or island in the sea type fibers. Such synthetic fibers may be formed via a spunbonding process or a meltblowing process. The acquisition layer facilitates the acquisition and the distribution of body exudates and may be placed between the topsheet and the absorbent layer. The acquisition layer may include cellulosic fibers.

The absorbent layers may be disposed in plurality in the absorbent core (62). Some portions of the absorbent layers may be configured to have substantially no absorbent material to form a channel or a plurality of channels. Channels may be useful for allowing the absorbent core (62) to bend upon swelling with fluids, such that the absorbent article conforms to the wearer's body after swelling and prevent sagging of the article. The channels may also be formed in the acquisition layer, and may be configured to at least partly match the channels of the absorbent layer in the thickness direction.

The elastic belt (40) of the article of the present invention acts to dynamically create fitment forces and to distribute the forces dynamically generated during wear. The front and back elastic belts (84, 86) may be joined with each other only at the side edges (89) to form side seams (32), a waist opening and two leg openings. Each leg opening may be provided with elasticity around the perimeter of the leg opening. The elasticity around the leg opening may be provided by the combination of elasticity from the front belt (84), the back belt (86), and the central chassis (38).

The longitudinal length of the backsheet and the outer cover layer (42) may be the same, or may be varied. For example, the outer cover layer (42) may have a shorter length compared to that of the backsheet, such that the outer cover layer (42) is devoid where the central chassis (38) overlaps the elastic belt (40). By such configuration, the elastic belt may have better breathability. Further, such configuration may provide cost saving. The transverse width of the backsheet and the outer cover layer (42) may be the same, or may be varied. For example, the backsheet may have a shorter transverse width compared to that of the outer cover layer (42). By such configuration, the longitudinal side edges (48) of the crotch panel (56), which make part of the leg openings, may have better breathability. Further, such configuration may provide cost saving.

The front elastic belt (84) and back elastic belt (86) are configured to impart elasticity to the belt (40). Referring to Figures 1B and 2, the front belt (84) and the back belt (86) may each comprise a laminate, the laminate comprising a plurality of elastic members (96) running in the transverse direction, an inner sheet (94), an outer sheet (92), and an outer sheet fold over (not shown) wherein the outer sheet fold over is an extension of the outer sheet material formed by folding the outer sheet material at the distal edge (88) of the front and back belts; wherein the belt elastic members (96) are sandwiched between two of these sheets. The longitudinal dimension between adjacent elastic members (96) form an elastic spacing. The front elastic belt (84) and the back elastic belt (86) may each be made only by elastic members (96), the inner sheet (94), the outer sheet (92), and the outer sheet fold over. The belt elastic members (96) may extend in the transverse direction to provide a ring like elastic belt (40) when the front elastic belt (84) and the back elastic belt (86) are joined. At least some of the elastic members (96) extend in the transverse direction substantially parallel to each other. All of the elastic members (96) may extend in the transverse direction substantially parallel to each other. Such an article may be economically made. The front and back elastic belt (84, 86) each may have transversely continuous proximal and distal edges, the proximal edge (90) being located closer than the distal edge (88) relative to the longitudinal center of the article. At least 10%, or at least from about 15% to not more than about 70%, of the front and back elastic belts from the waist opening in the longitudinal direction may be a laminate in active elasticity along the entire transverse dimension LW of the front and back elastic belts (84, 86). Referring to Figures 1B and 2, the front and back elastic belts (84, 86) may be treated such that certain regions are removed of its elastic activity to form a non-elastic region (221). For each front and back elastic belt (84, 86), the region overlapping the front and/or back waist panel (52, 54) of the central chassis (38) may be removed of its elastic activity and defining the non-elastic region (221).

The elastic belt region (40) may be closely associated with the function and quality of the article. Thus, materials for forming the elastic belt region (40), as well as the gathering profile of the elastic belt region, are carefully selected by the manufacturer to provide the desired tactile and visible senses. Tactile sense such as flexibility and cushiony touch may enhance perception of high quality. The appearance of gathers may intuitively connote the function of the article, or the function of the elastic belt region (40). For example, relatively big uniform gathers may connote a fluffy and soft feel. For example, a bubble kind of texture may connote a soft and cushiony feel. Further, other functions provided by the laminate such as stretchability for ease of application, comfort and softness, as well as breathability, may enhance the perception provided by the gather appearance. Gathers intentionally provided to have a certain appearance may intuitively communicate the functional benefits described above, and provide the favorable entire usage experience of the article by the user. The user may be the wearer or the caregiver.

The laminate having improved function intuitive appearance of the present invention may be made by selecting the material for making the laminate, and by providing the plurality of discrete bond units (234) in a certain arrangement, which is explained in further detail below. The material for providing the laminate may be selected from a lofty nonwoven material of relatively high basis weight for either the outer sheet (92) or the inner sheet (94), and provide the other of the outer sheet (92) and inner sheet (94) to have a difference in basis weight. Further, the laminate may be made by bonding the elastic members (96) in an appropriate denier, longitudinal pitch, and force; to one or both of the inner sheet (94) and the outer sheet (92).

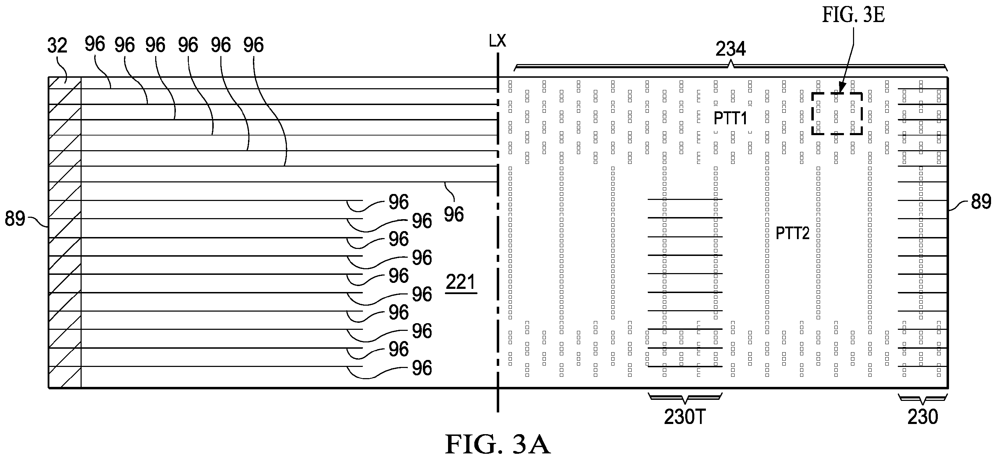

Referring to Figures 3A-3C, the laminate is made by bonding the elastic members (96) to at least one of the inner sheet (94) and the outer sheet (92), via a combination of an elastic bonding (230) and plurality of discrete bond units (234). In Figures 3A-3C, the laminate is shown with the elastic members (96) and elastic bonding (230) expressed in solid lines. In Figures 3A-3C, the plurality of discrete bond units (234) is only expressed in the right side of the front elastic belt (84), and the side seams (32) are shown in an unjoined state.

What is meant by elastic bonding (230) herein is a bonding that bonds the elastic member (96) along the side edges (89) of the front and back elastic belts (84, 86). Such elastic bonding is (230) may be provided by adhesive, heating, or ultrasound. The elastic bonding (230) is continuously applied to each elastic member (96) for a length of at least about 10mm, or from about 10mm to about 60 mm in the direction of stretch adjacent the side edges (89) of the front and back elastic belts (84, 86), including the length planned for side seaming. The elastic bonding (230) is to provide relatively strong bonding for the elastic member (96) and thus securely anchor the elastic member (96) within the laminate. The anchoring may be assisted by the side seaming. A certain percentage, or a greater percentage, of the dimension of the elastic bonding (230) along the side edges (89) may be seamed. The elastic bonding (230) may also be utilized for an effective process of deactivating a limited transverse dimension of the elastic member (96). Referring to Figures 2 and 3A-3C, the elastic member (96) may be deactivated in portions overlapping the absorbent core (62). In addition to the side edge regions, the elastic bonding (230T) may be provided on both sides of the certain transverse dimension of the elastic member (96) which is planned to be deactivated, wherein the portion of the elastic member between the elastic bondings (230T) are severed and deactivated. The deactivated portions of the elastic member is not shown in Figures 2 and 3A-3C. Such deactivation may be referred to herein as tummy cut, and the deactivated region may match the non-elastic region (221).

What is meant by plurality of discrete bond units (234) herein is a bonding applied to at least one of the inner sheet (94) and the outer sheet (92) for intermittently bonding the inner sheet (94) and the outer sheet (92). Such discrete bond unit (230) may be provided by adhesive, heating, or ultrasound. Each discrete bond unit has a longitudinal dimension of from about 0.5mm to about 20mm, preferably from about 0.5mm to about 6.0mm, and a transverse dimension of from about 0.5mm to about 6.0mm, preferably from about 0.5mm to about 2.0mm, wherein between any two discrete bond units, the discrete bond units have a longitudinal spacing of at least about 0.2mm with each other, and a transverse spacing of at least about 0.2mm with each other. All of the discrete bond units may be provided in the same longitudinal dimension and the same transverse dimension, respectively. Discrete bond units having different longitudinal and/or transverse dimensions may be used. The shape of the bond may be rectangular, circular, or oval.

The plurality of discrete bond units (234) are disposed such that there is at least one discrete bond unit disposed in each elastic spacing. By at least one discrete bond unit disposed in each elastic spacing, what is meant is that a discrete bond unit (234) in its complete longitudinal and transverse dimensions exists in the elastic spacing without contacting the elastic (96). For example, referring to Figure 3A, at least 2 discrete bond units (234) exist in each elastic spacing. By providing at least one discrete bond unit in each elastic spacing, the elastic members (96) are prevented from contacting each other. In that the elastic bonding (230) provides secure bonding of the elastic member (96) along the side seams (32), as well as the outer periphery of the non-elastic region (221), so long as there is at least one discrete bond unit (234) disposed in each elastic spacing, this prevents the elastic member (96) from moving away from its intended position. The plurality of discrete bond units (234) may also bond the elastic member (96) to at least one of the inner sheet (94) and the outer sheet (92). For an entire front elastic belt (84) or an entire back elastic belt (86), there may be no elastic member (96) bonded to the inner sheet (94) or the outer sheet (92) by a discrete bond unit (234). For an entire front elastic belt (84) or an entire back elastic belt (86), at least one to about 80% of the elastic members (96) may be bonded to the inner sheet (94) or the outer sheet (92) by a discrete bond unit (234). The plurality of discrete bond units (234) may only be provided to the outer sheet (92). The plurality of discrete bond units (234) may only be provided to the inner sheet (94). Referring to Figures 3A, the plurality of discrete bond units (234) may be provided for the entire area of the laminate. By providing the plurality of discrete bond units (234) for the entire area of the laminate, the plurality of discrete bond units (234) may serve as a bonding for the inner and outer sheets (92, 94) in regions where the elastic members (96) are severed. The plurality of discrete bond units (234) may be provided in regions adjacent the side edges (89) and thus overlapping the regions where the elastic bondings (230) are provided. Alternatively, the plurality of discrete bond units (234) may be provided only in regions where the elastic bondings (230) are not provided. The plurality of discrete bond units (234) may be provided at least in regions where the elastic member (96) is in active elasticity, wherein the elastic bondings (230) are devoid.

As mentioned above, all of the discrete bond units (234) may be provided in the same longitudinal dimension and the same transverse dimension, respectively. By providing each discrete bond unit in such way, and in a small enough dimension, various patterns may be created by the collection of discrete bond units.

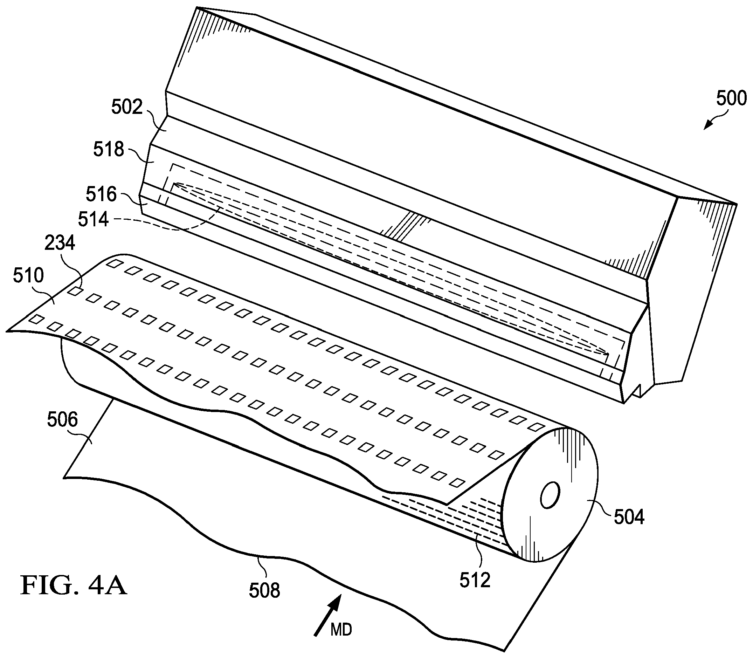

Figure 4A shows a perspective view of an embodiment of an apparatus (500) for applying hot melt adhesives to a sheet. The sheet may be either the outer sheet (92) or the inner sheet (94). A suitable apparatus for the present invention is described in PCT Publication WO2014/085063. The apparatus (500) includes a slot die applicator (502) and a sheet carrier (504). As shown in Figure 4A, a sheet (506) is advancing in a machine direction and is partially wrapped around the sheet carrier (504). More particularly, the sheet (506) includes a first surface (508) disposed opposite a second surface (510). And the first surface (508) of the sheet (506) is disposed on an outer surface (512) of the sheet carrier 504 while the second surface (510) of the sheet (506) advances past the slot die applicator (502). As discussed in more detail below, the second surface (510) of the sheet (506) advances past the slot die applicator (502) and adhesive is transferred from the slot die applicator (502) onto the second surface of the sheet in a pattern that is substantially the same as a pattern defined on the outer surface (512) of the sheet carrier (504). As discussed in more detail below, the sheet carrier (504) may be configured in various ways to deposit adhesive (530) discharged from a slot die applicator (502) onto a sheet (506) resulting in various different patterns, such as shown for example in Figures 3A-3G.

The slot die applicator (502) shown in Figure 4A is a generic representation of a device that is used to apply adhesive to the sheet (506). The slot die applicator may include a slot opening (514), a first lip (516), and a second lip (518). The first lip (516) may also be referred to herein as an upstream die lip, and the second lip (518) may also be referred to herein as a downstream die lip. The slot opening (514) is located between the first lip (516) and the second lip (518). Adhesive may be discharged from the slot opening (514) onto the second surface (510) of the sheet (506) as the sheet carrier (504) advances the sheet past the first lip (516), slot opening (514), and second lip (518) of the slot die applicator (502). The longitudinal dimension of the discrete bond units (234) are defined by the size in the CD direction of each slot opening (514). In Figure 4A, the slot opening (514) is shown as a series of small openings separated by spacings having a similar size as the openings. The sheet (506) is also intermittently compressed between the slot die applicator (502) and sheet carrier (504) as the sheet (506) advances past the slot die applicator (502). A suitable apparatus for the present invention is described in PCT Publication WO2014/085063. Various forms of slot die applicators may be used herein to apply adhesive to an advancing sheet according to methods and apparatuses. For example, U.S. Patent No. 7,056,386 provides a description of slot die applicators that may be used. Other examples of commercially available slot die applicators include Nordson Corporation's EP11 Series of Slot Die Applicators and ITW Dynatec Gmbh's APEX Series of Slot Die Auto Adhesive Applicators.

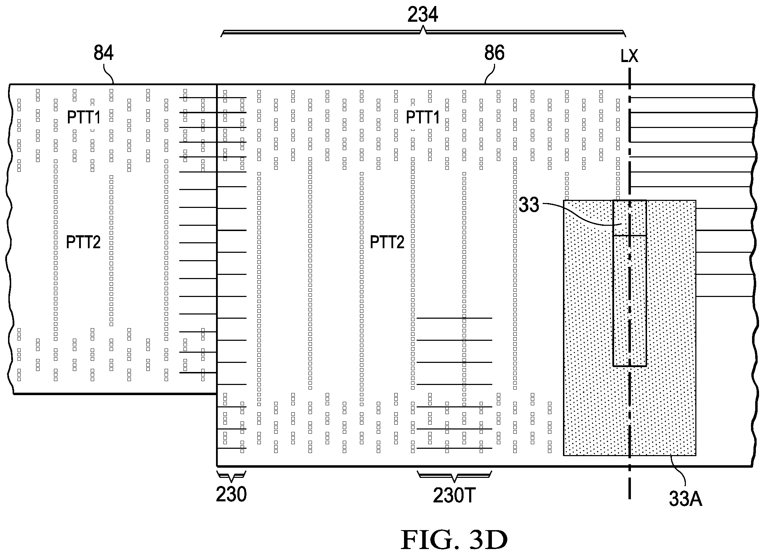

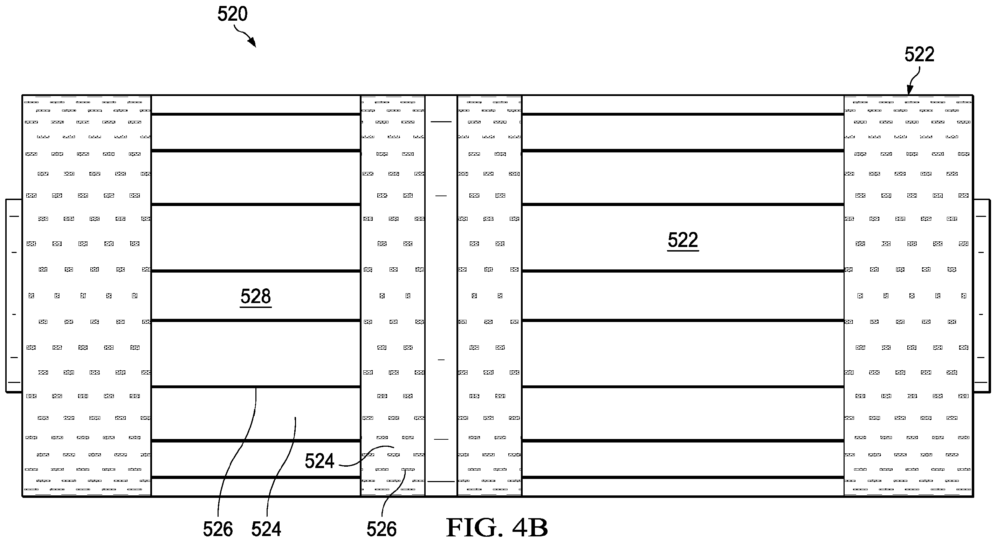

Figure 4B shows an embodiment of a sheet carrier (504) configured as a roller (520) adapted to advance a sheet (506) past the slot die applicator (502). The outer surface (512) of the sheet carrier (504) includes a plurality of pattern elements (522) that protrude radially outward from a base surface (524). Each pattern element (522) includes a pattern surface (526), and the radial protrusion of the pattern elements (522) from the base surface (524) define a distance, Hp, between the pattern surface (526) and the base surface (524). The base surface (524) is configured as a continuous surface (528), and the plurality of discrete pattern elements (522) are separated from each other by the continuous surface (528). The shape and size of the pattern surface (526) of each pattern element (522) may be identical or different from each other. As discussed in more detail below, as the sheet carrier (504) advances the sheet (506) past the slot die applicator (502), adhesive discharged from the slot die applicator is deposited onto the sheet in a pattern substantially matching the shapes of the pattern surfaces on the sheet carrier. The adhesive (530) is deposited onto the sheet (506) in discrete pattern areas (532) that correspond with and may mirror the shapes of the pattern surfaces (526) on the sheet carrier (504). Figure 4B shows an example pattern for providing the patterns shown in Figures 3A and 3D, wherein the front belt (84) and the back belt (86) are made simultaneously. The transverse dimension of the discrete bond units (234) are defined by the dimension of the pattern surface (526) in the circumferential direction.

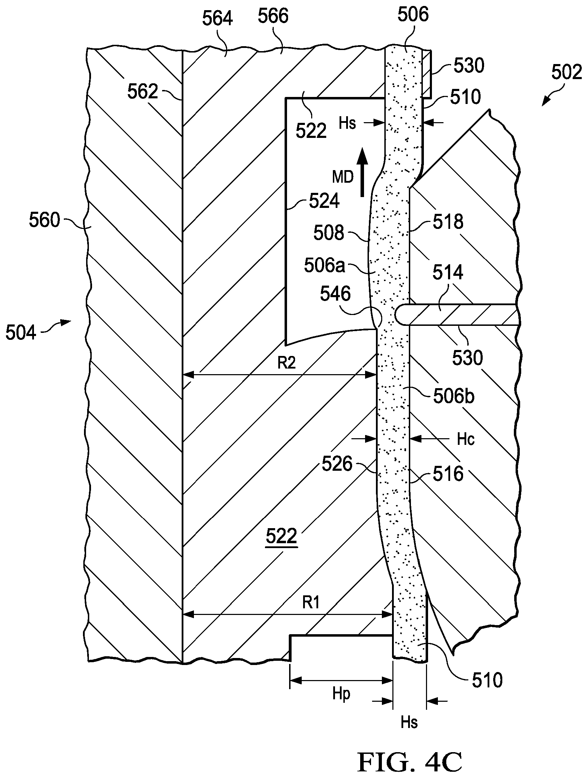

Figure 4C shows a detailed cross-sectional view of a sheet carrier (504) and a sheet (506) advancing past a slot die applicator (502). The sheet (506) has an unconstrained caliper, Hs, and has a first surface (508) disposed opposite of a second surface (510). The first surface (508) of the sheet (506) is disposed on the sheet carrier (504). And the sheet (506) and sheet carrier (504) are shown as advancing together in a machine direction, MD, past the slot die applicator (502). More particularly, the second surface (510) of the sheet (506) is advancing past a slot opening (514) located between an upstream lip (516) and a downstream lip (518) of the slot die applicator (502). As previously mentioned, the sheet carrier (504) is positioned adjacent the slot die applicator (502) to define a minimum distance, Hg, between the uncompressed pattern surface (526) of the pattern element (522) and the first lip (516) and the second lip (518) that is less than the unconstrained caliper, Hs, of the sheet (506). In addition, the sheet carrier (504) is positioned adjacent the slot die applicator (502) to define a minimum distance, Hb, between the base surface (524) and the first lip (516) and the second lip (518) that is greater than the unconstrained caliper, Hs, of the substrate. The apparatus (500) may also be configured such that a sum of the distance, Hp, and distance, Hg, is greater that the unconstrained caliper, Hs, of the sheet (506).

Thus, a portion (506a) of the sheet (506) that is located between the slot opening (514) of the slot die applicator (502) and the advancing base surface (524) is not pressed against the base surface (524). As such, although adhesive (530) is continuously discharged from the slot opening (514), adhesive (530) is not being transferred to the second surface (510) of the sheet (506).

Figure 4C further shows the sheet (506) wherein the base surface (524) has advanced past the slot opening (514) of the slot die applicator (502) such that a portion (506b) of the sheet (506) is between the first lip (516) of the slot die applicator (502) and a leading edge (546) of an advancing pattern surface (526). As previously discussed, the minimum distance, Hg, between the pattern surface (526) of the uncompressed pattern element (522) and the first lip (516) and the second lip (518) is less than the unconstrained caliper, Hs, of the sheet (506). As such, a portion (506b) of sheet (506) between the pattern surface (526) and the first lip (516) is pressed against and exerts forces on the pattern surface (526). Thus, the pattern element (522) and/or base surface (524) compresses, allowing the pattern surface (526) to deflect away from the first lip (516) to define a minimum distance, R2, between the pattern surface (526) and the non-compliant support surface (162). The adhesive (530) being discharged from the slot opening (514) is shown in Figure 6C as beginning to transfer to the second surface (510) of the sheet as the leading edge (546) of the pattern surface (526) and adjacent portion of the sheet (506) begin to advance past the slot opening (514).

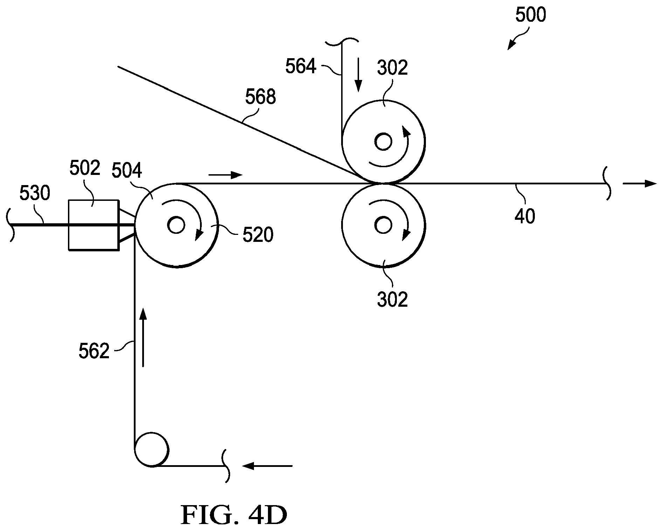

Figure 4D shows how a laminate of the present invention is assembled by a continuous length of outer sheet material (562), a continuous length of inner sheet material (564), and continuous elastic strands (568) combined to form a continuous elastic laminate. As discussed above, a fluid application apparatus (500) which includes a slot die applicator (502) and sheet carrier (504) may be used apply adhesive to one of the continuous length of outer sheet material (562), or a continuous length of inner sheet material (564), outer sheet material (562). Referring to Figure 4D, the continuous length of outer sheet material (562) is advanced in a machine direction onto a roller (520) and past a slot die applicator (502) where adhesive is applied, and then joined with the continuous elastic strands (568) and continuous length of inner sheet material (564) also advanced in a machine direction. Prior to joining with the continuous inner/outer sheet materials (562, 564), the elastic strands may be applied adhesive over a certain length in the machine direction for providing elastic bonding (230).

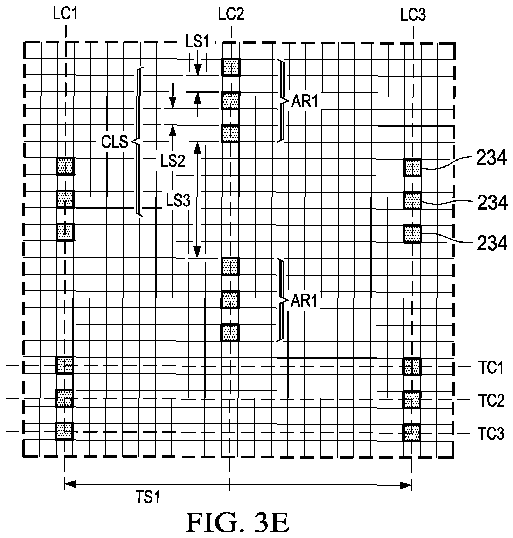

Referring back to Figures 3A and 3E, the plurality of discrete bond units (234) form a plurality of longitudinal columns (LC1, LC2, LC3...), which is a group of discrete bond units which fall on a same imaginary line extending parallel to the longitudinal axis. Each longitudinal column (LC) comprises a collection (CLS) of longitudinal spacings (LS1, LS2, LS3...). The plurality of discrete bond units (234) form a plurality of transverse columns (TC1, TC2, TC3...), which is a group of discrete bond units which fall on a same imaginary line extending parallel to the transverse axis. Each transverse column (TC) comprises a collection (CTS) of transverse spacings (TS1, TS2, TS3...). The collection of discrete bond units provided by at least 2 longitudinal columns and at least 2 transverse columns form a pattern of discrete bond units. The two dimensional pattern of discrete bond units may be made by more than 2 longitudinal columns and/or more than 2 transverse columns. The laminate of the present invention comprises at least 2 patterns of discrete bond units. What is meant by different pattern is a difference of discrete bond unit patterns in one or more of: the longitudinal dimension of the bond unit forming the pattern, the transverse dimension of the bond unit forming the pattern, the collection of longitudinal spacings (CLS), and the collection of transverse spacings (CTS).

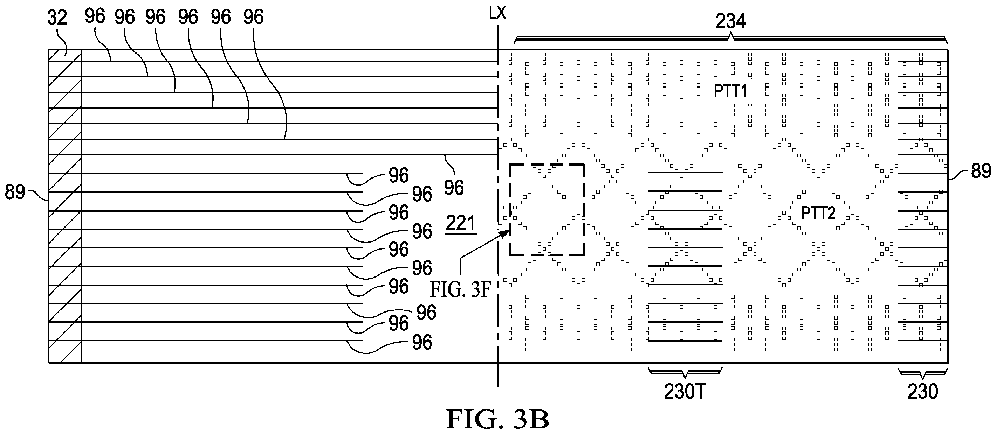

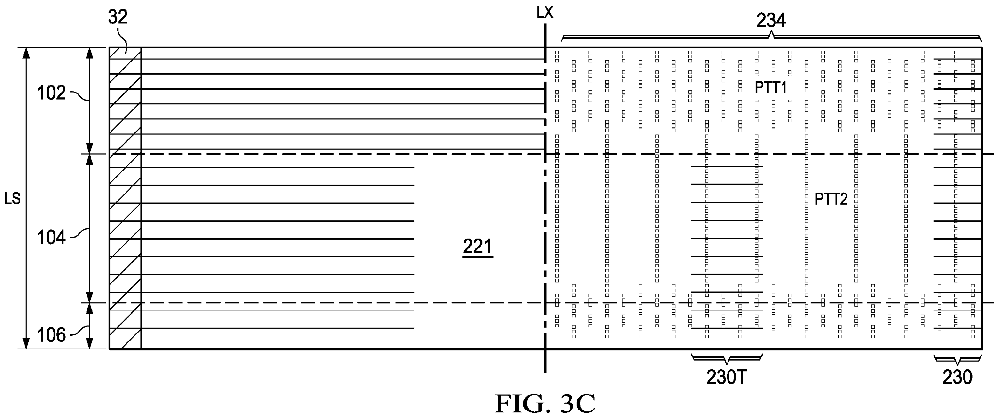

Referring to Figures 3A-3D, each laminate comprises a first pattern (PTT1) and a second pattern (PTT2) disposed adjacent to each other via a transverse border, wherein the patterns are different by at least the collection of transverse spacings (CTS). The first pattern (PTT1) is be provided adjacent the waist opening and adjacent the leg opening. The second pattern (PTT2) may be provided in the non-elastic region (221). Referring to Figures 3A and 3C, the first pattern (PTT1) adjacent the waist and leg openings has a smaller collection of transverse spacings (CTS) compared to that of the second pattern (PTT2). The transverse spacing of the second pattern is from about 1.2 to about 5 times of that of the first pattern. By providing the first and second patterns (PTT1, PTT2) in such way, the gathers provided by the first pattern (PTT1) may be provided more tightly disposed, thus connoting strength of the laminate at the waist/leg openings, while the gathers provided by the second pattern (PTT2) may be provided less tightly disposed, thus connoting softness and gentle fit in other regions of the laminate. Referring to Figure 3B, the first pattern (PTT1) adjacent the waist and leg openings and that of the second pattern (PTT2) may be provided in very different designs. By providing the first and second patterns in very different designs, the gathers resulting from the patterns may also appear very different. For example, the second pattern (PTT2) of Figure 3B may provide gathers appearing like bubbles. Referring to Figure 3D, the pattern of discrete bonding units of the front belt (84) and the back belt (86) may be coordinated for providing an undergarment like appearance. For example, the transverse border dividing the first pattern (PTT1) and the second pattern (PTT2) at the waist opening may be matched to provide a continuous band-like appearance at the waist opening. For example, the first pattern (PTT1) may be provided adjacent the leg opening of each of the front belt (84) and the back belt (86), but not necessary sharing a transverse border.

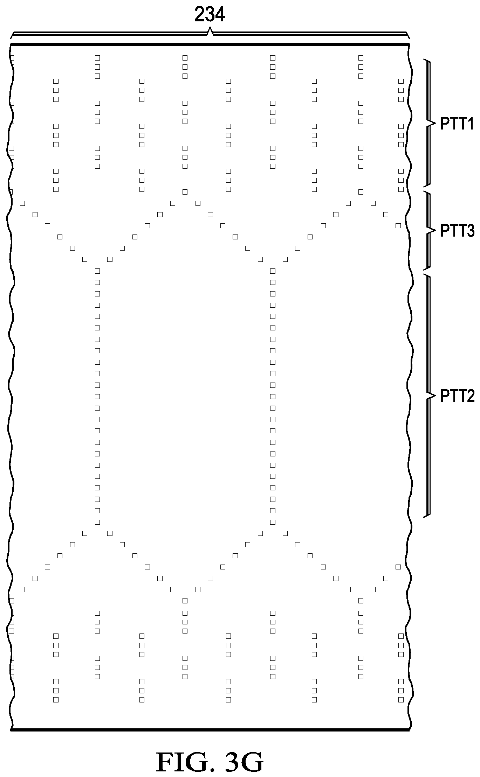

Referring to Figure 3G, the laminate may comprise a third pattern (PTT3) for dividing the first pattern (PTT1) and the second pattern (PTT2). The third pattern (PTT3) of Figure 3G may be provided in smaller collective longitudinal spacing and/or smaller collective transverse spacing in order to provide a boarder appearance, and further amplify the appearance difference of resulting gathers provided by the first and second patterns (PTT1, PTT2).

Further referring to Figures 3A - 3C, the elastic members (96) overlapping the first pattern (PTT1) may be disposed in a first pitch spaced apart in the longitudinal direction, and the elastic members (96) overlapping the second pattern (PTT2) may be disposed in a second pitch spaced apart in the longitudinal direction. By more or less matching the boarder of patterns of discrete bonding units (234) and pitch of elastic members (96), the resulting appearance of gathers may be further amplified.

While not shown, the 2 patterns of discrete bonding units may comprise a first pattern and a second pattern disposed adjacent to each other via a longitudinal border, wherein the patterns are different by at least the collection of longitudinal spacings. When the article comprises a front elastic belt (84), a back elastic belt (86), and a central chassis (38) bridging the front elastic belt and the back elastic belt, the first pattern may be provided on the region of the front and back elastic belt (84, 86) that does not overlap the central chassis (38).

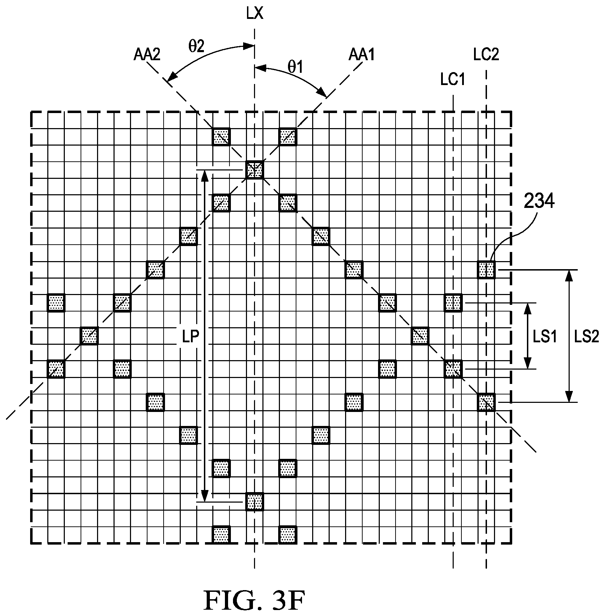

Referring to the first pattern (PTT1) of either Figure 3A or 3B and the second pattern (PTT2) of Figure 3B, there may be at least about 30%, preferably at least about 50%, of the collective longitudinal spacings (CLS) that are not constant. While not required, referring to Figure 3E and 3F, all of the longitudinal columns (LC) may have a non-constant collective spacing of discrete bond units. The collective longitudinal spacing (CLS) of any adjacent longitudinal columns (LC1, LC2) are different from each other. For example, longitudinal column LC1 and longitudinal column LC2 do not have the same collective longitudinal spacing (CLS).

Referring to Figure 3A and 3E, each of the plurality of longitudinal columns (LC) may comprise a first spacing and a second spacing, wherein the second spacing is greater than the first spacing, wherein a constant number of at least 2 discrete bond units (234) spaced apart with the first spacing form a first array (AR1), wherein the first array (AR1) is spaced apart with each other with the second spacing. From about 2 to about 10 discrete bond units (234) may form the first array (AR1), and the second spacing may be from about 3mm to about 15mm. When observing a plurality of longitudinal columns (LC1, LC2, LC3), the longitudinal position of the first array (AR1) may alternate in the transverse direction. Depending on the desired pattern of discrete bond units, the longitudinal columns (LC) may be spaced apart in the transverse direction at a pitch of from about 3mm to about 15mm. The pitch in the transverse direction of the longitudinal columns (LC) may be constant.

Referring to Figure 3B and 3F, the discrete bond units (234) across the plurality of longitudinal columns (LC) may be so configured to provide an angled alignment (AA1, AA2), the angled alignment being a linear or a curved continuation of a plurality of discrete bond units (234) across the plurality of longitudinal columns (LC). As in Figure 3F, the angled alignment (AA1, AA2) may be a linear continuation. The angled alignments (AA1, AA2) may have an angle of from about 30 degrees to about 60 degrees against the longitudinal axis, and in linear symmetry to the longitudinal axis with each other, and repeated at a constant pitch in the longitudinal direction to provide a pattern of repeating rhomboid shapes. The constant pitch in the longitudinal direction (LP) may be from about 6mm to about 25mm.

The aforementioned patterns of discrete bond units depicted in Figures 3A-3C may be planned to have the dimensions of Table 1 for providing the intended patterns.

**Table 1**

| | First Pattern Fig 3A, 3B | First Pattern Fig 3C | Second Pattern Fig 3A, 3C | Second Pattern Fig 3B |
|---|---|---|---|---|
| Longitudinal dimension (mm) | 1 | 1 | 1 | 1 |
| Transverse dimension (mm) | 1 | 1 | 1 | 1 |
| Transverse spacing (mm) | 13 | 6 | 20 | NA |
| Longitudinal (first) spacing (mm) | 1 | 1 | 1 | NA |
| Longitudinal second spacing (mm) | 6 | NA | NA | NA |
| Angle of angled alignment (degrees) | NA | NA | NA | 45 |
| Longitudinal pitch of pattern (mm) | NA | NA | NA | 28 |

As a result of the above mentioned configuration of the discrete bond units (234) as in Figures 3A-3C, the total bond area provided by the discrete bond units (234) may be controlled while maintaining integrity of the laminate, and further provide a zonal appearance of the laminate. The different zonal appearances may intuitively connote a certain function of the laminate as described above. Having a relatively small total bond area provided by the discrete bond units (234) results in less restriction for the inner sheet (94) and the outer sheet (92), as well as the inner and outer sheets (92. 94) against the elastic members (96). Without being bound by theory, it is believed that by having less restriction for the inner and outer sheet materials (92, 94) against the elastic members (96), this allows improved stretchability of the elastic members (96), which may provide ease of application.

Compared to elastic belts made only by elastic bonding (230) wherein all of the elastic members (96) are continuously bonded, the elastic belt (40) of the present invention may have a lower Stretch Circumference Force, according to the measurements herein. What is meant by Stretch Circumference Force is the loading force at a certain stretch level, which is believed to simulate initial stretch experience felt by the wearer or caregiver when inserting hands and stretch opening the article. Further, despite such relatively low Stretch Circumference Force, the elastic belt (40) of the present invention may maintain a suitable Fit Circumference Force, according to the measurements herein. What is meant by Fit Circumference Force is the unloading force at a certain stretch level, which is believed to simulate the force felt by the wearer while wearing the article. Accordingly, the article of the present invention has a Stretch Circumference Force of no more than about 6.5N, and a Fit Circumference Force of at least about 2.5N according to the measurements herein, wherein the ratio of the value of Stretch Circumference Force / Fit Circumference Force is less than about 2.5, preferably less than about 2.3. A relatively low value for the ratio of Stretch Circumference Force / Fit Circumference Force means that there is less difference between the loading and unloading force felt by the wearer, thus providing the sensorial qualities similar to that of an undergarment. Without being bound by theory, it is also believed that, by having less restriction for the inner and outer sheet materials (92, 94) against the elastic members (96), this improves the breathability of the overall laminate, which may enhance skin health. Without being bound by theory, it is also believed that the plurality of discrete bond units (234) provides a configuration wherein a greater percentage of the inner and outer sheet materials (92, 94) are available for forming the outer surfaces of the laminate when the elastic belt (40) is contracted, while the elastic members (96) remain positioned inside the thickness of the laminate. As such, the laminate is provided with improved loft and thickness, thus imparting improved comfort and softness when worn.

The bonding strength of the elastic bonding (230) and the plurality of discrete bond units (234) may be the same or may be varied. The elastic bonding (230) and the plurality of discrete bond units (234) may be provided in the same bonding method, such as by adhesive. The elastic bonding (230) and the plurality of discrete bond units (234) may be provided by the same hot melt adhesive. Exemplary hot melt adhesives suitable for the present invention include tradenames H4376 and H2401 available from Bostik.

Referring to Figure 3D, the article of the present invention may have a disposal tape (33) disposed on one of the front or back elastic region (84, 86) for assisting disposal of the article after use. The portion of the elastic region supporting the disposal tape (33) may be reinforced so that the pulling force generated when extending the disposal tape (33) does not damage the laminate. The elastic region superposing the disposal tape may be provided with a reinforcement adhesive (33A) disposed between the inner sheet (94) and the outer sheet (92).

Referring to Figure 2, for the belt-type pant, the proximal edges (90) of the front and back belt (84, 86) may be provided with an end seal in order to keep the inner and outer sheets (92, 94) closed at the proximal edges (90) and thus prevent elastic members (96) from being accessible. Such unaccessibility of elastic members (96) may be particularly advantageous when the article is for a young wearer. Alternatively or additionally, the elastic member (96) which is positioned closest to the proximal edge (90) may be provided with an elastic bonding (230) along the transverse dimension of the elastic member (96) in portions of active elasticity.

Referring to Figure 2, the elastic member (96) may be made by a plurality of elastic strands (96) running parallel to each other in the transverse direction, wherein the laminate has a region wherein the elastic strands (96) have a longitudinal pitch of from about 3mm to about 18mm, or from about 3mm to about 12mm, or from about 3mm to about 7mm.

The tensile stress (N/m) of the entirety of the front and back elastic belts (84, 86), respectively, may be profiled in order to provide the functional benefits of the present invention, such as ease of stretch and application, while also maintaining certain force during wear, to prevent the article from sagging after loading. When the elasticity of the front and back elastic belts (84, 86) are provided by a plurality of elastic members (96) running in the transverse direction, the tensile stress may be adjusted by one or more of the following methods; 1) elongation rate of the elastic member (96); 2) density (dtex) of the elastic member (96); 3) longitudinal pitch of multiple elastic members (96); and 4) effective length of elasticity of the elastic member (96) in the transverse direction. By elongation, "0% elongation" is meant the original length of the elastic member. When a portion of an elastic member (96) is removed of its elasticity, the remainder of the intact elastic member capable of imparting elasticity is defined as the "effective length of elasticity of an elastic member".

Referring to Figures 2 and 3C, the front and back elastic belts (26, 28) may each be divided into 3 zones spanning in the transverse direction and defined of its position from the distal edge (88) to the proximal edge (90) relative to the percentage of the seam length LS. In the example of Figure 2, the entirety of the length of the belt side edge (89) of the front region (26) is the front belt (84), and is seamed with a certain length of the belt side edge (89) of the back region (28) which is the back belt (86) to define a seam length LS. When seam length LS is considered 0% at the distal edge (88) and 100% at the proximal edge (90) of the side seam (32), the zones are defined as such: 0-35% is the waist zone (102), 35-85% is the tummy zone (104), and 85-100% is the leg zone (106). When there is an elastic member disposed at 35% from the distal edge (88), such elastic member is considered to be included in the waist zone (102). When there is an elastic member disposed at 85% from the distal edge (88), such elastic member is considered to be included in the tummy zone (104).

Certain zones of the belt may be disposed of elastic bodies having a density of no more than about 500dtex. Elastic bodies having a density of no more than about 500dtex may be disposed on one or more of the waist zone (102), or the leg zone (106). Elastic bodies having a density of no more than about 500dtex may be disposed on the tummy zone (104). At least 50% of the elastic members on each of the front belt (84) and the back belt (86) may have a density of no more than about 500dtex. Without being bound by theory, it is believed that elastic bodies of relatively low density impart an easy initial stretch experience when stretch opening the article (20), while maintaining a good fit during wear. Namely, use of such elastic bodies of relatively low density are advantageous in providing a controlled Stretch Circumference Force, while maintaining a certain Fit Circumference Force.

In the article of the present invention, the tensile stress of the back waist zone (102) may be provided higher than the front waist zone (102). The tensile stress of the back waist zone (102) may be provided at least about 10% higher than that of the front waist zone (102). The tensile stress of the front tummy zone (104) may be provided higher than the back tummy zone (104). The tensile stress of the front tummy zone (104) may be provided at least about 20% higher than that of the back tummy zone (104). The tensile stress of the back leg zone (106) may be provided significantly lower, preferably at least about 20% lower than that of the back tummy zone (104). The tensile stress of the front leg zone (106) may be provided significantly lower, preferably at least about 20% lower than that of the front tummy zone (104). Without being bound by theory, such profiling of the tensile stress per zone is believed to provide the article of the present invention with a shaped elastic belt (40) that conforms well to a human body, particularly to a lower torso of a child of less than 36 months of age, and therefore provide good fit and comfort to the wearer, without compromise of sagging prevention or leakage prevention.

For the belt-type pant, the longitudinal length LB of the back elastic belt (86) and the longitudinal length LF of the front elastic belt (84) may be provided the same, or the back elastic belt (86) may have a greater longitudinal length LB as in Figure 2. Referring to Figures 1B and 2, when the wearable article is assembled to form the waist opening and the leg openings, the wearable article (20) is folded along the transverse centerline TX such that the front distal edge (88) is aligned with the back distal edge (88). The front side edge (89) is also aligned with a portion of the back side edge (89). Then the front belt (84) and the back belt (86) are joined at the front and back side edges (89) at the seams (32). The front and back proximal edges (90), however, may not be aligned to one another. The back proximal edge (90) may be disposed longitudinally closer than the front proximal edge (90) relative to the transverse center line TX such that the proximal portion of the back side panel (82) extends toward the crotch panel (56) of the central chassis (38) beyond the front proximal edge (90). The side edge of the proximal portion of the back side panel (82) may not be joined to anywhere and free from attachment. Thus, the proximal portion of the back side panel (82) provides a buttock cover (95), as in Figure 1B.

The outer sheet (92) of the present invention may be a nonwoven having a basis weight of from about 10gsm to about 55gsm, or from about 10gsm to about 35gsm, and may have a fiber diameter of from about 0.8 dpf to about 6 dpf. The fiber diameter is described in denier per filament (dpf) used in the industry, which is grams / 9,000 meters of length of fiber. The outer sheet (92) nonwoven may be made by processes such as spunbond, spunlace, carded or air-laid; and may comprise fibers and/or filaments made of polypropylene (PP), polyethylene (PE), polyethylene phthalate (PET), polylactic acid/polylactide (PLA) or conjugate fibers (such as PE/PET, PE/PP, PE/PLA) as well as natural fibers such as cotton or regenerated cellulosic fibers such as viscose or lyocell. The outer sheet (92) nonwoven may be a multilayer or composite structure combining nonwovens made by different processes and fibers such as combining spunbond and carded nonwovens. The outer sheet (92) nonwoven may be made by biodegradable material, or derived from renewable resources. Exemplary material for the outer sheet (92) include: air-through carded nonwoven having a thickness of at least about 50µm, or at least about 80µm, or at least about 200µm. Such material may provide a soft lofty feeling to the garment-facing side. Suitable for the outer sheet (92) nonwoven of the present invention are air-through carded nonwoven material made of co-centric bicomponent fiber, crimping fiber made through core eccentric bicomponent filament or side by side bicomponent filament. Non-limiting examples of materials suitable for the outer sheet (92) nonwoven of the present invention include: 12-45gsm air-through carded nonwoven substrate comprising PE/PET bi-component fibers, such as those available from Beijing Dayuan Nonwoven Fabric Co. Ltd. or Xiamen Yanjan New Material Co. Ltd., and 8-45gsm spun melt nonwoven substrate comprising PP monofilament or PE/PP bi-component fibers, such as those available from Fibertex or Fitesa.

The inner sheet (94) of the present invention may be a nonwoven having a basis weight of from about 5gsm to about 45gsm, or from about 5gsm to about 35gsm. The inner sheet (94) nonwoven may have a fiber diameter of from about 0.5 dpf to about 4 dpf. The inner sheet (94) nonwoven may be made by processes such as spunbond, spunlace, carded or air-laid; and may comprise fibers and/or filaments made of polypropylene (PP), polyethylene (PE), polyethylene phthalate (PET), polylactic acid/polylactide (PLA) or conjugate fibers (such as PE/PET, PE/PP, PE/PLA) as well as natural fibers such as cotton or regenerated cellulosic fibers such as viscose or lyocell. The inner sheet (94) nonwoven may also be a multilayer or composite structure combining nonwovens made by different processes and fibers such as combining spunbond and carded nonwovens. The inner sheet (94) nonwoven may be made by biodegradable material, or derived from renewable resources. Non-limiting examples of materials suitable for the inner sheet (94) nonwoven of the present invention include: 12-30gsm air-through carded nonwoven substrate made of PE/PET bi-component staple fiber, such as those available from Beijing Dayuan Nonwoven Fabric Co. Ltd. or Xiamen Yanjan New Material Co. Ltd., and 8-30gsm spun melt nonwoven substrate comprising PP monofilament or PE/PP bi-component fibers, such as those available from Fibertex or Fitesa.

The basis weight of the outer sheet (92) and the inner sheet (94) may be adjusted such that the basis weight of the inner sheet (94) is not greater than the basis weight of the outer sheet (92). Thus, the outer sheet (92) may be provided with a soft lofty tactile sense which connotes high quality, while the inner sheet (94) may be kept thinner and conforming to the outer sheet (92), thus saving cost. Further, without being bound by theory, by providing the basis weight relationship as such, it is believed that skin sweating is effectively transported to the outer sheet (92) and outside the laminate, while preventing the transported sweat back to the inner sheet (94). The hydrophilicity/hydrophobicity of the outer sheet (92) and the inner sheet (94) may be adjusted such that the hydrophilicity of the outer sheet (92) is higher than that of the inner sheet (94). Without being bound by theory, it is believed that such gradient of hydrophilicity is advantageous in transporting skin sweat from the inner sheet (94) to the outer sheet (92) and outside the laminate. The inner sheet (94) nonwoven may be inherently hydrophobic. The inner sheet (94) nonwoven may be provided hydrophobicity by treating with hydrophobic melt additives into polymer resin in the fiber making process, or by applying hydrophobic additives after the nonwoven is formed. The outer sheet (92) nonwoven may inherently be hydrophobic, and thus provided relatively more hydrophilic than the inner sheet (94) by treating with hydrophilic melt additives into polymer resin in the fiber making process, or by applying hydrophilic additive after the nonwoven is formed.

The wearable article of the present invention may be provided color or have artwork provided on the materials for making the laminate. The region of the laminate superposing the first pattern (PTT1) may have a different color than the region of the laminate superposing the second pattern (PTT 1). Such color may have a brightness of higher than about 50 and a saturation of lower than about 80. Color of such brightness and saturation is believed to connote a soft perception.

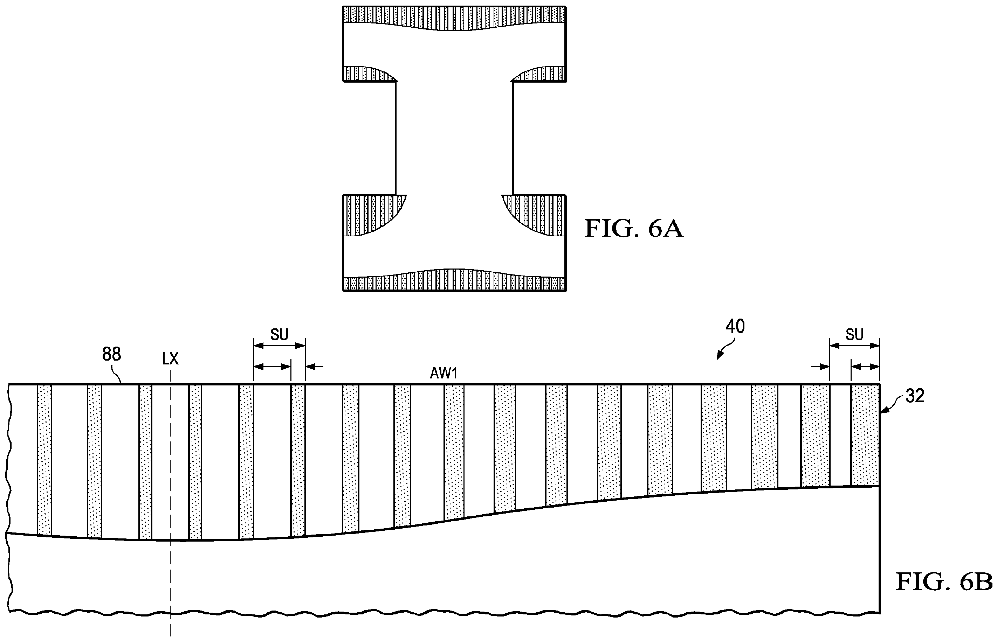

Referring to Figures 3A-3D and 6A-6B, the wearable article of the present invention may have the first pattern (PTT1) of discrete bond units provided adjacent the waist opening, wherein a first artwork (AW1) is provided on the laminate at least partly overlapping the first pattern (PTT1) adjacent the waist opening, wherein the first artwork (AW1) comprises a series of stripes in a first color having a longitudinal dimension and spaced apart with intervals in the transverse direction. The series of stripes may be in a constant transverse dimension and spacing, or may be in a non-constant transverse dimension and spacing. Referring to Figure 6B, the stripes of the first artwork (AW1) have a transverse spacing dimension which increases from the side seams (32) toward the transverse center of the laminate. For example, when considering one pair of stripe and spacing as a unit (SU), the transverse dimension of the stripe and spacing may be about 2:1 adjacent the side seam (32), wherein the dimension of the spacing within the unit gradually increases and replaces the dimension of the stripe, such that adjacent the transverse center, the transverse dimension of the stripe and spacing may be about 1:2. Such gradual increase of spacing within the unit from the side seam to the transverse center may take about 10 units of more. Further, the longitudinal dimension of the stripe adjacent the side seam (32) may be at least about 20mm, and gradually increase toward the transverse center, wherein the longitudinal dimension of the stripe adjacent the transverse center is at least about 10mm greater than that at the side seam (32), while being no greater than about 55mm. By providing the artwork adjacent the waist opening in such manner, this alleviates the sense of tightness at the waist opening.

Referring to Figure 6A, the first pattern (PTT1) may be further provided adjacent the leg opening, wherein a second artwork is provided on the laminate at least partly overlapping the first pattern (PTT1) adjacent the leg opening. The second artwork may be provided in the same first color as the first artwork. Unlike the first artwork, the longitudinal dimension of the second artwork may decrease from the side seam (32) toward the transverse center of the article.

Alternatively, the laminate superposing the first pattern (PTT1) provided along the waist opening may be devoid of color, namely have no added color on top of the materials for making the laminate. Such white color may connote underwear like appearance.

The wearable articles of the present invention may be provided in a flexible package wherein a certain number of wearable articles are packed together and compressed for making the package compact. While applying more compression force to the package may be beneficial for making the package as compact as possible, there may be a threshold in which the compression force affects the topography of the wearable article, such as gathers provided on the laminate. The package comprising the wearable articles of the present invention may have an In-Bag Compression of above 0% to about 30%. What is meant by "In-Bag Compression" as used herein is one minus the height of a stack of 10 diaper pads in millimeters, measured while under compression within a ply-bag, divided by the height of a stack of 10 diaper pads of the same type before compression, multiplied by 100. For example, if the height is the same before and after compression, In-Bag Compression is 0%.

The wearable articles of the present invention may be provided a first indicia for communicating a consumer benefit of the article, wherein the first indicia is further provided on the package.

### Whole Article Force Measurement

Force is measured using an Electronic Tensile Tester with a computer interface such as the MTS Criterion C42 running TestWorks 4 Software (available from MTS SYSTEMS (CHINA) CO., LTD) or equivalent instrument. A load cell is selected so that force results for the samples tested will be between 10 and 90% of capacity of the load cell used. The instrument is calibrated according to the manufacturer's instructions. All testing is performed in a room maintained at 23 ± 2 °C and 50 ± 5 % relative humidity.

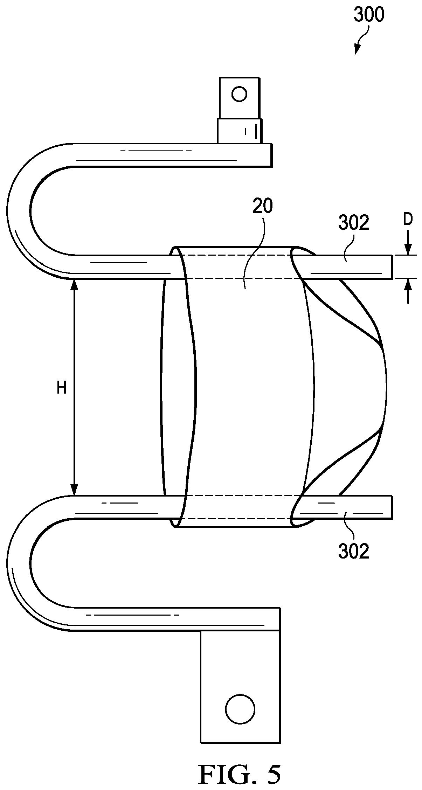

The tensile tester is fitted with hanger-type sample holding fixtures (300) as shown in Figure 5. Each fixture comprises a rigid linear rubber-coated horizontal bar section (302) to prevent sample slippage during testing. The outer bar diameter (including the rubber coating) of the horizontal bar sections is 10.0 mm. The central axes of the horizontal bar sections (302) are configured to remain parallel and in the same vertical plane throughout the test procedure. The gauge circumference is determined by the following equation:$Gauge Circumference = 2 \times \left(H+D+πD/2\right)$ where H is the vertical gap between the horizontal bar sections (302), and D is the outer diameter of the bar.

The instrument is set up to go through the following steps:

| | |
|---|---|
| Crosshead Speed | 254.0mm/min |
| Final Load Point | 19.61 N |
| Hold Time | 0 |
| Number of Cycles | 1 |
| Data Acquisition Rate | 50Hz |

An article (20) sample is inserted onto the upper horizontal bar section (302) so that the bar passes through the waist opening and one leg opening of the article. The crosshead is raised until the specimen hangs above the lower bar and does not touch lower bar (302). The load cell is tared and the crosshead is lowered to enable the lower bar (302) to be inserted through the waist opening and other leg opening without stretching the article. The article is adjusted so that the longitudinal centerline LX of the article is in a horizontal plane halfway between the upper and lower bars (302). The center of the side portion in contact with the bar (302) is situated on the same vertical axis as the instrument load cell. The crosshead is raised slowly while the article is held in place by hand as necessary until the force is between 0.05 and 0.1N, while taking care not to add any unnecessary force. The gauge circumference at this point is the Initial Gauge Circumference. The test is initiated and the crosshead moves up at 254 mm/min until a force of 19.6N is attained, then the crosshead immediately returns to the Initial Gauge Circumference at the same speed. The maximum circumference at 19.6N and the force at 70% of the maximum circumference during the loading segment and unloading segment of the test are recorded.

The maximum circumference (mm) at 19.6N is defined as the Full Stretch Circumference W1. The Full Stretch Circumference (mm) × 0.7 is defined as the 70% Stretch Circumference W2. The force (N) during the loading segment of the test at 70% Stretch Circumference is defined as the Stretch Circumference Force. The force (N) during the unloading segment of the test at 70% Stretch Circumference is defined as the Fit Circumference Force. Five samples are analyzed and their average are calculated and reported to the nearest 1mm or 0.01N, respectively.

### Belt Zone Tensile Stress Measurement

The tensile stress (N/m) is calculated by tensile force (N) divided by the specimen width (m). Force may be measured using an Electronic Tensile Tester with a computer interface such as the MTS Criterion C42 running TestWorks 4 Software (available from MTS SYSTEMS (CHINA) CO., LTD) or equivalent instrument. A load cell is chosen so that force results for the samples tested will be between 10 and 90% of capacity of the load cell. The instrument is calibrated according to the manufacturer's instructions. All testing is performed in a room maintained at 23 ± 2 °C and 50 ± 5 % relative humidity. The instrument is equipped with single line contact grips at least as wide as the test specimen.

To obtain test specimens, the sample article is cut open along the side seams (32), and the front and rear elastic belt sections (40) are removed from the central chassis (38) by separating the bonding between the waist belt and central chassis. Cold Spray may be used, paying attention not to make wrinkles in the belt sections. Care is taken not to spray on any belt elastic body (96). The obtained elastic belts (40) are severed into zones (102, 104, 106) according to the present invention with care not to cut any elastic body (96). Samples are pre-conditioned at 23 °C ± 2 C° and 50% ± 5% relative humidity for two hours prior to testing.

The instrument is set up to go through the following steps. Initial Gauge Length is calculated from the Initial Gauge Circumference which is determined during the Whole Article Force Test using separate identical articles, as described above. Initial Gauge Length = 0.5 Initial Gauge Circumference. The final gauge length is calculated from the Full Stretch Circumference which is determined during the Whole Article Force Test, as described above.

| | |
|---|---|
| Crosshead Speed | 254.0 mm/min |
| Data Acquisition Rate | 50Hz |
| Final Gauge Length | 0.5 × Full Stretch Circumference |
| Hold Time | 0 |
| Number of Cycles | 1 |

One end of the specimen is clamped into the upper clamp and the load is tared. The other end of the specimen is clamped into the lower clamp. Approximately 5 mm of each end of the specimen is behind the contact line of the grip. The test is started and the specimen is extended to the final gauge length at a crosshead speed of 254 mm/min, then immediately returned to the original gauge length at the same speed. The specimen is extended in the article transverse direction during the test. The unload force at 70% of the Final Gauge Length during the unload segments of the test is recorded.

Five articles are analyzed and the unload forces are recorded for each of the front and back zones (102, 104, 106). The average tensile force (N) is calculated to the nearest 0.01 N for each zone including the front and back specimens for that zone. The tensile stress for each zone is calculated by the average tensile force (N) divided by the average longitudinal length (m) and reported to the nearest 0.1 N/m.

### EXAMPLES

Examples 1-3 and Comparative Examples 1-4 were obtained as such and subject to the tests described below.

Example 1: Size 4 (L-size) belt-type pant article having the configuration, elastic bonding, and pattern of discrete bond units of Figure 3A, and Table 1 above, and elastic profile and other properties of Table 2 below.

Example 2: Size 4 (L-size) belt-type pant article having the configuration elastic bonding, and pattern of discrete bond units of Figure 3B and Table 1 above, and elastic profile and other properties of Table 2 below.

Example 3: Size 4 (L-size) belt-type pant article having the configuration, elastic bonding, and pattern of discrete bond units of Figure 3C, and Table 1 above, and elastic profile and other properties of Table 2 below.

Comparative Example 1: Same structural configuration and elastic bonding as "Ichiban Pants" Size 4 available in PRC from P&G, however, replacing the outer sheet with material that are the same as Examples 1-3.

Comparative Example 2: "Baby Care Royal Weak Acid" Size 4 (lot # 20210402), purchased in July 2021 in PRC.

Comparative Example 3: "Huggies Penguin" Size 4 (lot # 20210412) purchased in July 2021 in PRC.

Comparative Example 4: "Goo.N Angel" Size 4 (lot # 20201202) purchased in July 2021 in PRC.

**Table 2**

| Zone | Examples 1-3 | Tensile Stress (N/m) |
|---|---|---|
| | Dtex / elongation / Number of elastics | |
| Front waist zone | 470Dtex / 180% / 7 | 18.9 |
| Front tummy zone | 940Dtex / 190% / 8 with tummy cut (*1) | 16.0 |
| Front leg zone | 470Dtex / 180% / 2 with tummy cut (*1) | 3.8 |
| Back waist zone | 470Dtex / 180% / 4 | 22.5 |
| | 940Dtex/ 130% / 3 | |
| Back tummy zone | 940Dtex/ 130% / 1 | 11.3 |
| | 470Dtex / 180% /4 | |
| | 470Dtex / 240% / 1with tummy cut (*1) | |
| Back leg zone | 470Dtex / 240% / 2 with tummy cut (*1) | 6.7 |

| | | |
|---|---|---|
| (*1) "Tummy cut" in Table 2 refers to deactivation of elasticity at the transverse central area of elastic strands resulting in 68% effective length of elasticity. | | |

### 1. Technical Measurements

The Waist Circumference Forces were measured according to the "Whole Article Force Measurement" method herein and results provided in Table 3 below. The tensile stress per zone were measured according to the "Belt Zone Tensile Stress Force Measurement" method herein and results provided in Table 2 above.

**Table 3**

| | Examples 1-3 | Comparative Example 1 | Comparative Example 2 | Comparative Example 3 | Comparative Example 4 |
|---|---|---|---|---|---|
| Stretch Circumference Force (N) | 6.1 | 6.3 | 5.8 | 7.54 | 7.8 |
| Fit Circumference Force (N) | 3.1 | 2.8 | 3.0 | 3.84 | 3.9 |

### 2. Consumer Acceptance Test

60 panelists who were caregivers of babies using Size 4 (L size) pant diapers and having a mixture of usage experience of major brands of similar price range used in the test were recruited. There were about equal number of caregivers of boy and girl babies in the age group of 25-36 months old. Two rounds of tests were conducted for a total 7 finished product test samples.

In the first round test, 5 finished product test samples (Example 1 and Comparative Examples 1-4 ) were provided to the panelists to touch and feel with their hands one by one. Each respondent was asked to fill in a questionnaire individually after touching the test sample one by one. In the questionnaire, there were 5 values as found in Table 4, and each respondent was requested to sort and rate the test samples against those values using the ratings from 1 to 10, which were scored as such: '1= poor, 10 = excellent'. The scores were averaged . And lastly each respondent was requested to rank the tested samples against the values using the rating from 1 to 5, which were scored as such:' 1= best, 5 = worst'. The scores were averaged. The results for the first round test are summarized in Table 4.

**Table 4**

| Values | Example 1 | Comparative Example 1 | Comparative Example 2 | Comparative Example 3 | Comparative Example 4 |
|---|---|---|---|---|---|
| | | | | | |
| Overall rating | 75(*3) | 73(*3) | 58 | 53 | 52 |
| Functional Intuitive Appearance | 79(*2) | 69 | 66 | 57 | 54 |
| Belt softness | 83(*2) | 75 | 75 | 72 | 70 |
| Comfort fit around waist | 79(*3) | 75(*3) | 67 | 69 | 71 |
| Pajama comfort | 78(*3) | 77(*3) | 65 | 59 | 60 |
| Overall Ranking | 2(*2) | 2.5 | 3.3 | 3.6 | 3.7 |

| | | | | | |
|---|---|---|---|---|---|
| (*2) These scores were statistically significantly better against Comparative Examples 1-4 at 90% confidence level. (*3) These scores were statistically significantly better against Comparative Examples 2-4 at 90% confidence level. | | | | | |

According to the test results in Tables 3 and 4, Example 1 which meets the requirements of the present invention has statistically significantly higher "Function intuitive appearance" and "Belt softness" and "Overall ranking" compared to Comparative Examples 1-4, and being statistically significantly better in "Overall rating", "Comfort fit around waist" "Pajama comfort" compared to Comparative Example 2-4, while having favorable Stretch Circumference Force and Fit Circumference Force values. It is believed that the gathers provided by the unique pattern of discrete bond units of Example 1 provides an improved function intuitive appearance and belt quality, which affected the consumer's perception of distinctiveness and the belt softness and comfort fit.

In the second round test, 4 finished product test samples including Example 1-2 and Comparative Example 2 were tested. Each respondent was asked to fill in a questionnaire individually after touching the test sample one by one. In the questionnaire, there were 3 values as found in Table 5, and each respondent was requested to sort and rate the test samples against those values using the ratings from 1 to 10, which were scored as such: '1= poor, 10 = excellent'. The scores were averaged. The results for the second round test are summarized in Table 5.

**Table 5**

| Values | Example 1 | Example 2 | Comparative Example 2 |
|---|---|---|---|
| Overall rating | 73 | 69 | 55 |
| Functional Intuitive Appearance | 72 | 69 | 44 |
| Belt quality | 74 | 71 | 63 |

According to the test results in Tables 3 and 5, Examples 1-2 which meet the requirements of the present invention have statistically significantly higher "Overall rating" "Functional intuitive appearance" and "Belt quality" compared to Comparative Example 2. It is believed that the gathers provided by the unique pattern of discrete bond units of Example 1-2 provides an improved function intuitive appearance and belt softness, which affected the consumer's perception of distinctiveness and the belt quality.

## Claims

1. A wearable article (20) continuous in a longitudinal direction and a transverse direction comprising a front elastic belt region (84), a back elastic belt region (86), a crotch region (30), and a pair of side seams (32) which join the front elastic belt region (84) and the back elastic belt region (86) to form a waist opening and a pair of leg openings; the crotch region (30) extending longitudinally between the front elastic belt region (84) and the back elastic belt region(86);
wherein each of the front and back elastic belt region (86) comprises a laminate comprising an inner sheet (94), an outer sheet (92), and a plurality of elastic members (96) running in the transverse direction, the longitudinal dimension between adjacent elastic members (96) forming an elastic spacing;
wherein the laminate further comprises an elastic bonding (230) which continuously bonds the elastic members (96) for at least about 10mm in the direction of stretch in a region adjacent the side edges (89) of the front and back elastic belt regions (86), and a plurality of discrete bond units (234) disposed between the elastic bondings (230) in the transverse direction, each discrete bond unit (234) applied to at least one of the inner sheet (94) and the outer sheet (92), wherein there is at least one discrete bond unit (234) disposed in each elastic spacing,
the plurality of discrete bond units (234) forms a plurality of longitudinal columns (LC1, LC2, LC3), each longitudinal column (LC) comprising a collection of longitudinal spacings (LS1, LS2, LS3);
the plurality of discrete bond units (234) forms a plurality of transverse columns (TC1, TC2, TC3), each transverse column (TC) comprising a collection of transverse spacings (TS1, TS2, TS3),
wherein a collection of discrete bond units (234) provided by at least 2 longitudinal columns (LC) and at least 2 transverse columns (TC) form a pattern of discrete bond units (234), wherein the laminate comprises at least a first pattern (PTT1) and a second pattern (PTT2) of discrete bond units (234), the first pattern (PTT1) and the second pattern (PTT2) of discrete bond units (234) having a difference in one or more of:
the longitudinal dimension, the transverse dimension, the collection of longitudinal spacings (LS1, LS2, LS3), and the collection of transverse spacings (TS1, TS2, TS3); and
wherein the first pattern (PTT1) and the second pattern (PTT2) are disposed adjacent to each other via a transverse border, wherein the patterns are different by at least the collection of transverse spacings (TS1, TS2, TS3); and
wherein the first pattern (PTT1) is provided adjacent the waist opening and adjacent the leg opening; and
wherein the first pattern (PTT1) adjacent the waist and leg openings has a smaller collection of transverse spacings (CTS) compared to that of the second pattern (PTT2), with the transverse spacing of the second pattern being from 1.2 to 5 times of that of the first pattern.

2. The wearable article (20) of Claim 1 wherein each discrete bond unit (234) has a longitudinal dimension of from about 0.5mm to about 20mm, and a transverse dimension of from about 0.5mm to about 6.0mm.

3. The wearable article (20) of Claim 1 or 2 wherein between any two discrete bond units (234), the discrete bond units (234) have a longitudinal spacing of at least about 0.2mm with each other and a transverse spacing of at least about 0.2mm with each other; and wherein all of the discrete bond units (234) are provided in the same longitudinal dimension, and the same transverse dimension, respectively.

4. The wearable article (20) of any of Claims 1-3 wherein the first pattern (PTT1) and the second pattern (PTT2) disposed adjacent to each other via a longitudinal border, wherein the patterns are different by at least the collection of longitudinal spacings (LS1, LS2, LS3...).

5. The wearable article (20) of Claim 4 comprising a front elastic belt (84), a back elastic belt (86), and a central chassis (38) bridging the front elastic belt and the back elastic belt (84, 86), wherein the first pattern (PTT1) is provided on the region of the front and back elastic belt (84, 86) that does not overlap the central chassis (38).

6. The wearable article (20) of any of the preceding claims wherein the laminate superposing the first pattern (PTT1) has a different color than the laminate superposing the second pattern (PTT2).

7. The wearable article (20) of any of the preceding claims wherein the first pattern (PTT1) is provided adjacent the waist opening, a first artwork (AW1) is provided on the laminate at least partly overlapping the first pattern (PTT1) adjacent the waist opening, wherein the first artwork (AW 1) extends the entire transverse dimension of the laminate and comprises a first color, the first artwork (AW1) having a first longitudinal dimension at the side seam (32) and a second longitudinal dimension along the longitudinal axis, wherein the second longitudinal dimension is greater than the first longitudinal dimension.

8. The wearable article (20) of Claim 7 wherein the first artwork (AW1) comprises a plurality of stripes in the first color, wherein the stripes have a transverse spacing dimension which increases from the side seam (32) toward the transverse center of the laminate.

9. The wearable article (20) of Claim 7 or 8 wherein the first pattern (PTT1) is further provided adjacent the leg opening, a second artwork (AW2) is provided on the laminate at least partly overlapping the first pattern (PTT1) adjacent the leg opening, wherein the second artwork (AW2) comprises the first color, the second artwork (AW2) having a third longitudinal dimension at the side seam (32), wherein the longitudinal dimension of the second artwork (AW2) decreases from the third longitudinal dimension towards the transverse center.

10. The wearable article (20) of Claim 1 wherein the first pattern (PTT1) is provided along the waist opening, wherein the laminate superposing the first pattern (PTT1) is devoid of color.

11. The wearable article (20) of any of the preceding claims further comprising a disposal tape (33) disposed on one of the front elastic belt region (84) and the back elastic belt region (86), wherein the elastic belt region (40) superposing the disposal tape (33) is provided with a reinforcement adhesive disposed between the inner sheet (94) and the outer sheet (92).

12. A package comprising a plurality of the wearable articles (20) of any of the preceding claims wherein the package has an In-Bag Compression of above 0%to about 30%, as measured according to the test method set out herein.

13. A package comprising a plurality of wearable articles (20) of any of the preceding claims wherein the article is provided a first indicia for communicating a consumer benefit of the wearable article (20), wherein the first indicia is further provided on the package.

14. A method of making the pattern of discrete bond units (234) of the wearable article (20) of any of Claims 1-16 by applying an adhesive discharged from a slot die applicator (502) to at least one of the inner sheet (94) and the outer sheet (92) in the pattern of discrete bond units (234), the slot die applicator (502) including a slot opening (514), a first lip (516), and a second lip (518), the slot opening (514) located between the first lip (516) and the second lip (518), and the sheet (506) having a first surface (508) disposed opposite of a second surface (510) and an unconstrained caliper, Hs, the method comprising the steps of:
continuously advancing the sheet (506) in a machine direction;
engaging the sheet (506) with a substrate carrier roller (520), the roller (520) comprising a non-compliant support surface (162) and a pattern element (522), the pattern element (522) including a pattern surface (526) wherein the pattern element (522) extends away from the non-compliant support surface (162) to define a first minimum distance, R1, between the pattern surface (526) and the non-compliant support surface (162);
positioning the roller (520) adjacent the slot die applicator (502) to define a minimum distance, Hg, between the pattern surface (526) of the pattern element (522) and the first lip (516) and the second lip (518) that is less than the unconstrained caliper, Hs, of the sheet (506);
advancing the second surface (510) of the sheet (506) past the slot die applicator (502) while the first surface (508) of the sheet (506) is disposed on the roller (520);
intermittently deflecting the pattern surface (526) toward the non-compliant support surface (162) such to define a second minimum distance, R2, between the pattern surface (526) and the non-compliant surface (162), wherein R2 is less than R1, by advancing the sheet (506) and the pattern element (522) past the first lip (516), the slot opening (514), and the second lip (518) of the slot die applicator (502) while the first surface (508) of the sheet (506) is disposed on the roller (520), and
discharging adhesive from the slot opening (514) of the slot die applicator (502) onto the second surface (510) of the sheet (506).

## Patentansprüche

1. Am Körper tragbarer Artikel (20), ununterbrochen in einer Längsrichtung und einer Querrichtung, umfassend einen vorderseitigen Gummibandgürtelbereich (84), einen rückseitigen Gummibandgürtelbereich (86), einen Schrittbereich (30) und ein Paar Seitennähte (32), die den vorderseitigen Gummibandgürtelbereich (84) und den rückseitigen Gummibandgürtelbereich (86) verbinden, um eine Taillenöffnung und ein Paar Beinöffnungen zu bilden; wobei sich der Schrittbereich (30) zwischen dem vorderseitigen Gummibandgürtelbereich (84) und dem rückseitigen Gummibandgürtelbereich (86) der Länge nach erstreckt;
wobei jeder des vorderseitigen und des rückseitigen Gummibandgürtelbereichs (86) ein Laminat umfasst, umfassend eine innere Lage (94), eine äußere Lage (92) und eine Vielzahl von Gummibandelementen (96), die in der Querrichtung verlaufen, wobei die Längsabmessung zwischen aneinander angrenzenden Gummibandelementen (96) einen elastischen Zwischenraum bildet;
wobei das Laminat ferner eine elastische Bindung (230), die die Gummibandelemente (96) für mindestens etwa 10 mm in der Dehnungsrichtung in einem Bereich angrenzend an die Seitenkanten (89) der vorderseitigen und rückseitigen Gummibandgürtelbereiche (86) ununterbrochen verbindet, und eine Vielzahl von separaten Bindungseinheiten (234) umfasst, die zwischen den elastischen Bindungen (230) in der Querrichtung angeordnet sind, wobei jede separate Bindungseinheit (234) auf mindestens eine der inneren Lage (94) und der äußeren Lage (92) aufgebracht ist, wobei mindestens eine separate Bindungseinheit (234) in jedem elastischen Zwischenraum angeordnet ist,
die Vielzahl von separaten Bindungseinheiten (234) eine Vielzahl von Längsspalten (LC1, LC2, LC3) bildet, wobei jede Längsspalte (LC) eine Ansammlung von Längszwischenräumen (LS1, LS2, LS3) umfasst;
die Vielzahl separater Bindungseinheiten (234) eine Vielzahl von Querspalten (TC1, TC2, TC3) bildet, wobei jede Querspalte (TC) eine Ansammlung von Querzwischenräumen (TS1, TS2, TS3) umfasst,
wobei eine Ansammlung von separaten Bindungseinheiten (234), die durch mindestens 2 Längsspalten (LC) und mindestens 2 Querspalten (TC) bereitgestellt wird, ein Muster aus separaten Bindungseinheiten (234) bildet, wobei das Laminat mindestens ein erstes Muster (PTT1) und ein zweites Muster (PTT2) aus separaten Bindungseinheiten (234) umfasst, wobei das erste Muster (PTT1) und das zweite Muster (PTT2) aus separaten Bindungseinheiten (234) einen Unterschied in einem oder mehreren der Folgenden aufweisen: der Längsabmessung, der Querabmessung, der Ansammlung von Längszwischenräumen (LS1, LS2, LS3) und der Ansammlung von Querzwischenräumen (TS1, TS2, TS3); und
wobei das erste Muster (PTT1) und das zweite Muster (PTT2) über einen Querrand angrenzend aneinander angeordnet sind, wobei sich die Muster durch mindestens die Ansammlung von Querzwischenräumen (TS1, TS2, TS3) unterscheiden; und
wobei das erste Muster (PTT1) angrenzend an die Taillenöffnung und angrenzend an die Beinöffnung bereitgestellt ist; und
wobei das erste Muster (PTT1) angrenzend an den Taillen- und Beinöffnungen eine kleinere Ansammlung von Querzwischenräumen (CTS) im Vergleich zu der des zweiten Musters (PTT2) aufweist, wobei der Querzwischenraum des zweiten Musters das 1,2- bis 5-Fache desjenigen des ersten Musters beträgt.

2. Am Körper tragbarer Artikel (20) nach Anspruch 1, wobei jede separate Bindungseinheit (234) eine Längsabmessung von etwa 0,5 mm bis etwa 20 mm und eine Querabmessung von etwa 0,5 mm bis etwa 6,0 mm aufweist.

3. Am Körper tragbarer Artikel (20) nach Anspruch 1 oder 2, wobei zwischen beliebigen zwei separaten Bindungseinheiten (234) die separaten Bindungseinheiten (234) einen Längszwischenraum von mindestens etwa 0,2 mm zueinander und einen Querzwischenraum von mindestens etwa 0,2 mm zueinander aufweisen; und wobei alle separaten Bindungseinheiten (234) jeweils in derselben Längsabmessung und derselben Querabmessung bereitgestellt sind.

4. Am Körper tragbarer Artikel (20) nach einem der Ansprüche 1 bis 3, wobei das erste Muster (PTT1) und das zweite Muster (PTT2) über einen Längsrand aneinander angrenzend angeordnet sind, wobei sich die Muster durch mindestens die Ansammlung von Längszwischenräumen (LS1, LS2, LS3...) unterscheiden.

5. Am Körper tragbarer Artikel (20) nach Anspruch 4, umfassend einen vorderseitigen Gummibandgürtel (84), einen rückseitigen Gummibandgürtel (86) und eine zentrale Grundeinheit (38), die den vorderseitigen Gummibandgürtel und den rückseitigen Gummibandgürtel (84, 86) überbrückt, wobei das erste Muster (PTT1) auf dem Bereich des vorderseitigen und rückseitigen Gummibandgürtels (84, 86), der die zentrale Grundeinheit (38) nicht überlappt, bereitgestellt ist.

6. Am Körper tragbarer Artikel (20) nach einem der vorstehenden Ansprüche, wobei das Laminat, das das erste Muster (PTT1) überlagert, eine andere Farbe aufweist als das Laminat, das das zweite Muster (PTT2) überlagert.

7. Am Körper tragbarer Artikel (20) nach einem der vorstehenden Ansprüche, wobei das erste Muster (PTT1) angrenzend an die Taillenöffnung bereitgestellt ist, eine erste Grafik (AW1) auf dem Laminat bereitgestellt ist, die das erste Muster (PTT1) angrenzend an die Taillenöffnung wenigstens teilweise überlappt, wobei sich die erste Grafik (AW1) über die gesamte Querabmessung des Laminats erstreckt und eine erste Farbe umfasst, wobei die erste Grafik (AW1) eine erste Längsabmessung an der Seitennaht (32) und eine zweite Längsabmessung entlang der Längsachse aufweist, wobei die zweite Längsabmessung größer als die erste Längsabmessung ist.

8. Am Körper tragbarer Artikel (20) nach Anspruch 7, wobei die erste Grafik (AW1) eine Vielzahl von Streifen in der ersten Farbe umfasst, wobei die Streifen eine Querzwischenraumabmessung aufweisen, die von der Seitennaht (32) in Richtung der Quermitte des Laminats zunimmt.

9. Am Körper tragbarer Artikel (20) nach Anspruch 7 oder 8, wobei das erste Muster (PTT1) ferner angrenzend an die Beinöffnung bereitgestellt ist, eine zweite Grafik (AW2) auf dem Laminat bereitgestellt ist, die wenigstens teilweise das erste Muster (PTT1) angrenzend an die Beinöffnung überlagert, wobei die zweite Grafik (AW2) die erste Farbe umfasst, wobei die zweite Grafik (AW2) eine dritte Längsabmessung an der Seitennaht (32) aufweist, wobei die Längsabmessung der zweiten Grafik (AW2) von der dritten Längsabmessung in Richtung der Quermitte abnimmt.

10. Am Körper tragbarer Artikel (20) nach Anspruch 1, wobei das erste Muster (PTT1) entlang der Taillenöffnung bereitgestellt ist, wobei das Laminat, das das erste Muster (PTT1) überlagert, frei von Farbe ist.

11. Am Körper tragbarer Artikel (20) nach einem der vorstehenden Ansprüche, ferner umfassend ein Entsorgungsband (33), das auf einem von dem vorderseitigen Gummibandgürtelbereich (84) und dem rückseitigen Gummibandgürtelbereich (86) angeordnet ist, wobei der Gummibandgürtelbereich (40), der das Entsorgungsband (33) überlagert, mit einem Verstärkungsklebstoff bereitgestellt wird, der zwischen der inneren Lage (94) und der äußeren Lage (92) angeordnet ist.

12. Verpackung, umfassend eine Vielzahl von am Körper tragbaren Artikeln (20) nach einem der vorstehenden Ansprüche, wobei die Verpackung eine Stapelhöhe innerhalb des Beutels von über 0 % bis etwa 30 % nach dem hier beschriebenen Prüfverfahren aufweist.

13. Verpackung, umfassend eine Vielzahl von am Körper tragbaren Artikeln (20) nach einem der vorstehenden Ansprüche, wobei dem Artikel eine erste Markierung zum Kommunizieren eines Verbrauchernutzens des am Körper tragbaren Artikels (20) bereitgestellt ist, wobei die erste Markierung ferner auf der Verpackung bereitgestellt ist.

14. Verfahren zur Herstellung des Musters aus separaten Bindungseinheiten (234) des tragbaren Artikels (20) nach einem der Ansprüche 1 bis 16 durch Aufbringen eines Klebstoffs, der aus einem Schlitzdüsenapplikator (502) abgegeben wird, auf mindestens eine der inneren Lage (94) und der äußeren Lage (92) in dem Muster aus separaten Bindungseinheiten (234), wobei der Schlitzdüsenapplikator (502) eine Schlitzöffnung (514), eine erste Lippe (516) und eine zweite Lippe (518) einschließt, wobei die Schlitzöffnung (514) zwischen der ersten Lippe (516) und der zweiten Lippe (518) angeordnet ist, und wobei die Lage (506) eine erste Oberfläche (508), die gegenüberliegend einer zweiten Oberfläche (510) angeordnet ist, und eine unbelastete Dicke, Hs, aufweist, das Verfahren umfassend die Schritte:
ununterbrochenes Vorwärtsbewegen der Lage (506) in einer Maschinenlaufrichtung;
Ineingriffbringen der Lage (506) mit einer Substratträgerwalze (520), wobei die Walze (520) Folgendes umfasst: eine nicht nachgiebige Trägeroberfläche (162) und ein Musterelement (522), wobei das Musterelement (522) eine Musteroberfläche (526) einschließt, wobei sich das Musterelement (522) von der nicht nachgiebigen Trägeroberfläche (162) weg erstreckt, um einen ersten Mindestabstand R1 zwischen der Musteroberfläche (526) und der nicht nachgiebigen Trägeroberfläche (162) zu definieren;
Anordnen der Walze (520) benachbart zu dem Schlitzdüsenapplikator (502), um einen Mindestabstand, Hg, zwischen der Musteroberfläche (526) des Musterelements (522) und der ersten Lippe (516) und der zweiten Lippe (518) zu definieren, der kleiner ist als die nicht zusammengepresste Dicke, Hs, der Lage (506);
Vorwärtsbewegen der zweiten Oberfläche (510) der Lage (506) an dem Schlitzdüsenapplikator (502) vorbei, während die erste Oberfläche (508) der Lage (506) auf der Walze (520) angeordnet ist;
unterbrochenes Ablenken der Musteroberfläche (526) zu der nicht nachgiebigen Trägeroberfläche (162) hin derart, dass ein zweiter Mindestabstand, R2, zwischen der Musteroberfläche (526) und der nicht nachgiebigen Oberfläche (162) bestimmt wird, wobei R2 kleiner ist als R1, durch Vorwärtsbewegen der Lage (506) und des Musterelements (522) an der ersten Lippe (516), der Schlitzöffnung (514) und der zweiten Lippe (518) des Schlitzdüsenapplikators (502) vorbei, während die erste Oberfläche (508) der Lage (506) auf der Walze (520) angeordnet ist; und
Abgeben von Klebstoff aus der Schlitzöffnung (514) des Schlitzdüsenapplikators (502) auf die zweite Oberfläche (510) der Lage (506).

## Revendications

1. Article portable (20) continu dans une direction longitudinale et une direction transversale comprenant une région de ceinture élastique avant (84), une région de ceinture élastique arrière (86), une région d'entrejambe (30), et une paire de coutures latérales (32) qui joignent la région de ceinture élastique avant (84) et la région de ceinture élastique arrière (86) pour former une ouverture de taille et une paire d'ouvertures de jambe ; la région d'entrejambe (30) s'étendant longitudinalement entre la région de ceinture élastique avant (84) et la région de ceinture élastique arrière (86) ;
dans lequel chacune de la région de ceinture élastique avant et arrière (86) comprend un stratifié comprenant une feuille interne (94), une feuille externe (92), et une pluralité d'éléments élastiques (96) se prolongeant dans la direction transversale, la dimension longitudinale entre des éléments élastiques (96) adjacents formant un espacement élastique ;
dans lequel le stratifié comprend en outre une liaison élastique (230) qui lie en continu les éléments élastiques (96) sur au moins environ 10 mm dans la direction d'étirement dans une région adjacente aux bords latéraux (89) des régions de ceinture élastique avant et arrière (86), et une pluralité d'unités de liaison distinctes (234) disposées entre les liaisons élastiques (230) dans la direction transversale, chaque unité de liaison distincte (234) appliquée à au moins l'une parmi la feuille interne (94) et la feuille externe (92), dans lequel il y a au moins une unité de liaison distincte (234) disposée dans chaque espacement élastique,
la pluralité d'unités de liaison distinctes (234) forme une pluralité de colonnes longitudinales (LC1, LC2, LC3), chaque colonne longitudinale (LC) comprenant un ensemble d'espacements longitudinaux (LS1, LS2, LS3) ;
la pluralité d'unités de liaison distinctes (234) forme une pluralité de colonnes transversales (TC1, TC2, TC3), chaque colonne transversale (TC) comprenant un ensemble d'espacements transversaux (TS1, TS2, TS3),
dans lequel un ensemble d'unités de liaison distinctes (234) fourni par au moins 2 colonnes longitudinales (LC) et au moins 2 colonnes transversales (TC) forme un motif d'unités de liaison distinctes (234), dans lequel le stratifié comprend au moins un premier motif (PTT1) et un second motif (PTT2) d'unités de liaison distinctes (234), le premier motif (PTT1) et le second motif (PTT2) d'unités de liaison distinctes (234) ayant une différence dans un ou plusieurs parmi : la dimension longitudinale, la dimension transversale, l'ensemble d'espacements longitudinaux (LS1, LS2, LS3), et l'ensemble d'espacements transversaux (TS1, TS2, TS3) ; et
dans lequel le premier motif (PTT1) et le second motif (PTT2) sont disposés adjacents l'un à l'autre par l'intermédiaire d'une bordure transversale, dans lequel les motifs sont différents par au moins l'ensemble d'espacements transversaux (TS1, TS2, TS3) ; et
dans lequel le premier motif (PTT1) est fourni adjacent à l'ouverture de taille et adjacent à l'ouverture de jambe ; et
dans lequel le premier motif (PTT1) adjacent aux ouvertures de taille et de jambe a un ensemble plus petit d'espacements transversaux (CTS) par comparaison avec celui du second motif (PTT2), avec l'espacement transversal du second motif étant de 1,2 à 5 fois celui du premier motif.

2. Article portable (20) selon la revendication 1 dans lequel chaque unité de liaison distincte (234) a une dimension longitudinale allant d'environ 0,5 mm à environ 20 mm, et une dimension transversale allant d'environ 0,5 mm à environ 6,0 mm.

3. Article portable (20) selon la revendication 1 ou 2 dans lequel entre deux unités de liaison distinctes (234) quelconques, les unités de liaison distinctes (234) ont un espacement longitudinal d'au moins environ 0,2 mm l'une par rapport à l'autre et un espacement transversal d'au moins environ 0,2 mm l'une par rapport à l'autre ; et dans lequel toutes les unités de liaison distinctes (234) sont fournies dans la même dimension longitudinale, et la même dimension transversale, respectivement.

4. Article portable (20) selon l'une quelconque des revendications 1 à 3 dans lequel le premier motif (PTT1) et le second motif (PTT2) sont disposés adjacents l'un à l'autre par l'intermédiaire d'une bordure longitudinale, dans lequel les motifs sont différents par au moins l'ensemble d'espacements longitudinaux (LS1, LS2, LS3...).

5. Article portable (20) selon la revendication 4 comprenant une ceinture élastique avant (84), une ceinture élastique arrière (86), et un châssis central (38) pontant la ceinture élastique avant et la ceinture élastique arrière (84, 86), dans lequel le premier motif (PTT1) est fourni sur la région de la ceinture élastique avant et arrière (84, 86) qui ne chevauche pas le châssis central (38).

6. Article portable (20) selon l'une quelconque des revendications précédentes dans lequel le stratifié superposant le premier motif (PTT1) a une couleur différente du stratifié superposant le second motif (PTT2).

7. Article portable (20) selon l'une quelconque des revendications précédentes dans lequel le premier motif (PTT1) est fourni adjacent à l'ouverture de taille, un premier dessin (AW1) est fourni sur le stratifié chevauchant au moins partiellement le premier motif (PTT1) adjacent à l'ouverture de taille, dans lequel le premier dessin (AW1) s'étend sur toute la dimension transversale du stratifié et comprend une première couleur, le premier dessin (AW1) ayant une première dimension longitudinale au niveau de la couture latérale (32) et une deuxième dimension longitudinale le long de l'axe longitudinal, dans lequel la deuxième dimension longitudinale est supérieure à la première dimension longitudinale.

8. Article portable (20) selon la revendication 7 dans lequel le premier dessin (AW1) comprend une pluralité de bandes dans la première couleur, dans lequel les bandes ont une dimension d'espacement transversal qui augmente de la couture latérale (32) vers le centre transversal du stratifié.

9. Article portable (20) selon la revendication 7 ou 8 dans lequel le premier motif (PTT1) est en outre fourni adjacent à l'ouverture de jambe, un second dessin (AW2) est fourni sur le stratifié chevauchant au moins partiellement le premier motif (PTT1) adjacent à l'ouverture de jambe, dans lequel le second dessin (AW2) comprend la première couleur, le second dessin (AW2) ayant une troisième dimension longitudinale au niveau de la couture latérale (32), dans lequel la dimension longitudinale du second dessin (AW2) diminue de la troisième dimension longitudinale vers le centre transversal.

10. Article portable (20) selon la revendication 1 dans lequel le premier motif (PTT1) est fourni le long de l'ouverture de taille, dans lequel le stratifié superposant le premier motif (PTT1) est dépourvu de couleur.

11. Article portable (20) selon l'une quelconque des revendications précédentes comprenant en outre un ruban jetable (33) disposé sur l'une de la région de ceinture élastique avant (84) et de la région de ceinture élastique arrière (86), dans lequel la région de ceinture élastique (40) superposant le ruban jetable (33) est pourvue d'un adhésif de renforcement disposé entre la feuille interne (94) et la feuille externe (92).

12. Conditionnement comprenant une pluralité d'articles portables (20) selon l'une quelconque des revendications précédentes dans lequel le conditionnement a une compression en sachet de plus de 0 % à environ 30 %, telle que mesurée selon le procédé de test décrit ici.

13. Conditionnement comprenant une pluralité d'articles portables (20) selon l'une quelconque des revendications précédentes dans lequel l'article est muni d'un premier repère pour communiquer un avantage consommateur de l'article portable (20), dans lequel le premier repère est en outre fourni sur le conditionnement.

14. Procédé de fabrication du motif d'unités de liaison distinctes (234) de l'article portable (20) selon l'une quelconque des revendications 1 à 16 en appliquant un adhésif déchargé à partir d'un applicateur à filière plate (502) sur au moins l'une parmi la feuille interne (94) et la feuille externe (92) dans le motif d'unités de liaison distinctes (234), l'applicateur à filière plate (502) comportant une ouverture d'encoche (514), une première lèvre (516), et une seconde lèvre (518), l'ouverture d'encoche (514) étant située entre la première lèvre (516) et la seconde lèvre (518), et la feuille (506) ayant une première surface (508) disposée à l'opposé d'une seconde surface (510) et une épaisseur sans contrainte, Hs, le procédé comprenant les étapes consistant à :
faire avancer en continu la feuille (506) dans une direction de la machine ;
mettre en prise la feuille (506) avec un rouleau porteur de substrat (520), le rouleau (520) comprenant une surface de support non souple (162) et un élément de motif (522), l'élément de motif (522) comportant une surface de motif (526) dans lequel l'élément de motif (522) s'étend à l'écart de la surface de support non souple (162) pour définir une première distance minimale, R1, entre la surface de motif (526) et la surface de support non souple (162) ;
positionner le rouleau (520) adjacent à l'applicateur à filière plate (502) pour définir une distance minimale, Hg, entre la surface de motif (526) de l'élément de motif (522) et la première lèvre (516) et la seconde lèvre (518) qui est inférieure à l'épaisseur sans contrainte, Hs, de la feuille (506) ;
faire avancer la seconde surface (510) de la feuille (506) au-delà de l'applicateur à filière plate (502) alors que la première surface (508) de la feuille (506) est disposée sur le rouleau (520) ;
dévier de façon intermittente la surface de motif (526) vers la surface de support non souple (162) de façon à définir une seconde distance minimale, R2, entre la surface de motif (526) et la surface non souple (162), dans lequel R2 est inférieure à R1, en faisant avancer la feuille (506) et l'élément de motif (522) au-delà de la première lèvre (516), l'ouverture d'encoche (514), et la seconde lèvre (518) de l'applicateur à filière plate (502) alors que la première surface (508) de la feuille (506) est disposée sur le rouleau (520), et
décharger l'adhésif à partir de l'ouverture d'encoche (514) de l'applicateur à filière plate (502) sur la seconde surface (510) de la feuille (506).
